(19) Europäisches Patentamt
European Patent Office
Office européen des brevets

(11) **EP 4 755 347 A1**

(12) **EUROPEAN PATENT APPLICATION**
published in accordance with Art. 153(4) EPC

(43) Date of publication:
**10.06.2026 Bulletin 2026/24**

(21) Application number: **24848228.3**

(22) Date of filing: **29.07.2024**

(51) International Patent Classification (IPC):
***A61F 2/24*** (2006.01)

(52) Cooperative Patent Classification (CPC):
**A61F 2/24**

(86) International application number:
**PCT/CN2024/108211**

(87) International publication number:
**WO 2025/026273 (06.02.2025 Gazette 2025/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.07.2023 CN 202310954713**
**28.07.2023 CN 202310945501**

(71) Applicant: **Biotyx Medical (Shenzhen) Co., Ltd.**
**Shenzhen, Guangdong 518000 (CN)**

(72) Inventors:
• **FU, Wenchao**
**Shenzhen, Guangdong 518000 (CN)**
• **ZHANG, Gui**
**Shenzhen, Guangdong 518000 (CN)**
• **ZHANG, Deyuan**
**Shenzhen, Guangdong 518000 (CN)**
• **WANG, Xinlin**
**Shenzhen, Guangdong 518000 (CN)**
• **HU, Yinming**
**Shenzhen, Guangdong 518000 (CN)**

(74) Representative: **Prinz & Partner mbB**
**Patent- und Rechtsanwälte**
**Rundfunkplatz 2**
**80335 München (DE)**

(54) **DEGRADABLE VALVE STENT, VALVE STENT FOR PULMONARY ARTERIAL VALVE, PULMONARY ARTERIAL VALVE, AND HEART VALVE**

(57) The present application belongs to the technical field of interventional medical instruments, and particularly relates to a degradable valve stent, a valve stent for a pulmonary valve, a pulmonary valve, and a heart valve. The degradable valve stent includes a stent body which is constructed in a preset shape, and a proximal body and a distal body that are respectively connected to two axial ends of the stent body. The stent body, the proximal body, and the distal body overall form a circumferentially closed ringlike structure. Each of the stent body, the proximal body, and the distal body is at least partially degradable, so that the circumferentially closed ringlike structure of the degradable valve stent can be deconstructed. The heart valve includes a valve leaflet and the degradable valve stent. The valve leaflet is connected to the degradable valve stent. The strength of a radial support force of the degradable valve stent provided in the present application meets an application requirement. Moreover, the degradable valve stent is degradable, and can be disintegrated, so that the circumferentially closed ringlike structure can be deconstructed and does not cause difficulties and obstacles for subsequent implantation, thereby better meeting a usage requirement.

EP 4 755 347 A1

**FIG. 5**

## Description

This application claims priority to Chinese Patent Application No. 202310945501.6, filed with the China National Intellectual Property Administration on July 28, 2023 and entitled "Degradable Valve Stent and Heart Valve", and Chinese Patent Application No. 202310954713.0, filed with the China National Intellectual Property Administration on July 28, 2023 and entitled "Valve Stent for Pulmonary Valve and Pulmonary Valve", which are incorporated herein by reference in their entirety.

## Technical Field

The present application belongs to the technical field of interventional medical instruments, and particularly relates to a degradable valve stent, a valve stent for a pulmonary valve, a pulmonary valve, and a heart valve.

## Background Art

Heart valve disease is a common heart disease including congenital heart disease and acquired heart disease. The congenital heart disease means that valvular lesions are detected at birth, while the acquired heart disease arises from gradual heart valve damage over years of constant opening and closing, leading to valvular stenosis or insufficiency. This results in impaired blood flow or valvular regurgitation. With the growing aging population, senile valvular heart disease as well as valve lesions caused by coronary artery disease and myocardial infarction have become increasingly common. At present, common treatment methods for these diseases include valve replacement implemented by surgical operation or minimally invasive transcatheter implantation. Since it is very hard for current technology to suture an external valve to a cardiac valve annulus and maintain a relatively good closure effect, in the two currently common treatment methods, a surgical valve or an implanted valve is positioned and fixed through a valve stent, and is then placed at a corresponding position by surgical operation or implantation, to solve the problems of stenosis or insufficiency of a diseased valve.

At present, valve stents on commercially available valves are basically made of non-degradable materials such as 316L stainless steel, nickel-titanium alloy, and cobalt-chromium alloy. When these materials remain in a human body as foreign bodies for a long time, they easily cause excessive intimal hyperplasia, resulting in vascular mid-to-late restenosis, chronic inflammation, late and very late thrombosis, and also leading to difficulty in reimplantation. Particularly for patients with congenital valve diseases, as they grow older, the hearts gradually become enlarged, and the sizes of valves need to be adapted. If a valve stent of a pre-implanted valve is non-deconstructible ("deconstruction" means that a valve stent is circumferentially separated and is no longer a closed annular structure, so that it loses its radial supporting capacity and fails to restrict a range it can support radially), the re-implantation will inevitably be restricted subsequently. This brings significant difficulty in subsequent implantation and poses numerous potential hazards for the patients. Therefore, it is necessary to develop an absorbable valve stent that can play a role in supporting while being gradually degraded and deconstructed or even being absorbed by an organism until it completely disappears, thereby avoiding corresponding safety hazards due to the presence of a plurality of valve stents in a body.

## Summary of the Invention

The present application aims to overcome the above technical problems, and provides a degradable valve stent, a valve stent for a pulmonary valve, a pulmonary valve, and a heart valve.

A first aspect of the present application provides a degradable valve stent, including a stent body which is constructed in a preset shape, and a proximal body and a distal body that are respectively connected to two axial ends of the stent body. The stent body, the proximal body, and the distal body overall form a circumferentially closed ringlike structure. Each of the stent body, the proximal body, and the distal body is at least partially degradable, so that the circumferentially closed ringlike structure of the degradable valve stent is deconstructed. The degradable valve stent of the present application has good mechanical properties, can provide effective support, has a short degradation cycle because of use of fewer materials, and meets the requirements of not limiting valve stent reimplantation and avoiding use of a plurality of valve stents in a body.

A second aspect of the present application provides a valve stent for a pulmonary valve, including a tubular stent body, and a proximal body and a distal body that are respectively connected to two axial ends of the stent body. The stent body includes a plurality of porous grid structures. The distal body includes a plurality of unit bodies, each of which has an "M"-shaped contour, where m unit bodies are included, and m is a natural number greater than or equal to 2. The valve stent for the pulmonary valve of the present application can meet the requirements of the pulmonary valve, can expand uniformly, and has good usage reliability.

A third aspect of the present application provides a valve stent for a pulmonary valve, including the degradable valve stent described above. The distal body includes a plurality of unit bodies, each of which has an "M"-shaped contour, where m unit bodies are included, and m is a natural number greater than or equal to 2. The valve stent for the pulmonary valve of the present application has good mechanical properties, can provide effective support, has a short degradation cycle because of the use of fewer materials, meets the requirements of not limiting valve stent reimplantation and avoiding use of a plurality of valve stents in a body, can meet the requirements of the pulmonary valve, can expand uniformly, and has good usage reliability.

A fourth aspect of the present application provides a pulmonary valve, including a valve leaflet and the valve stent for a pulmonary valve described above. The valve leaflet is connected to the valve stent for a pulmonary valve.

A fifth aspect of the present application provides a heart valve, including a valve leaflet and the degradable valve stent described above. The valve leaflet is connected to the degradable valve stent.

The present application provides a degradable valve stent and a heart valve. The degradable valve stent includes a stent body, a proximal body, and a distal body. The proximal body and the distal end are respectively connected to two axial ends of the stent. The stent body, the proximal body, and the distal body overall form a circumferential closed ringlike structure, and each of the stent body, the proximal body, and the distal body is at least partially degradable. In this way, the degradable valve stent of this structure not only has the degradability itself, but also can be disintegrated and absorbed within preset time, so that the circumferentially closed ringlike structure of the valve stent is deconstructed, and the restriction on a radial supporting range is lost, so that the valve stent does not bring difficulties and obstacles for subsequent implantation. Moreover, the entire degradable valve stent also has relatively good supporting performance, and the strength of the radial support force meets the application requirement. Thus, by using the degradable valve stent, the heart valve can be effectively supported, and the degradable valve stent can be degraded. No restriction will be imposed on reimplantation. Usage requirements for the support performance and the degradability of the stent are well met.

The embodiments of the present application provide a valve stent for a pulmonary valve and a pulmonary valve. The valve stent includes a stent body, a proximal body, and a distal body. The proximal body and the distal body are respectively connected to two ends of the stent body. The stent body is provided with a plurality of porous grid structure. The distal body is configured as a plurality of unit bodies, each of which has an "M"-shaped contour. The unit bodies are connected to the stent body. In this way, in the expansion process of the entire valve stent, since the "M"-shaped unit bodies can pull connection points and constrain expansion paths, and the porous grid structures play a role in pulling and constraining during the expansion, the entire valve stent can uniformly expand and has good consistency in the overall shape. By using the valve stent, the pulmonary valve can uniformly expand, properly meets the usage requirement for uniform expansion of the pulmonary valve, and has good reliability.

## Brief Description of the Drawings

Various other advantages and benefits will become apparent to those of ordinary skill in the art upon reading the following detailed description of the preferred implementations. The drawings are for the purpose of illustrating the preferred implementations only and are not to be considered as a limitation on the present application. Furthermore, the same components are denoted by the same reference numerals throughout the drawings.

FIG. 1 is a schematic structural diagram of a degradable valve stent after expansion according to an embodiment of the present application;

FIG. 2 is a schematic diagram of a planar structure of a degradable valve stent in a compressed state according to an embodiment of the present application;

FIG. 3 is a schematic diagram of a planar structure of a degradable valve stent in a compressed state according to an embodiment of the present application;

FIG. 4 is a schematic structural diagram of a second kind of degradable valve stent after expansion according to an embodiment of the present application;

FIG. 5 is an exploded diagram of a degradable valve stent after expansion according to an embodiment of the present application;

FIG. 6 is a schematic structural diagram of a second kind of distal body in a degradable valve stent according to an embodiment of the present application;

FIG. 7 is a schematic structural diagram of a third kind of distal body in a degradable valve stent according to an embodiment of the present application;

FIG. 8 is a schematic structural diagram of a unit body according to an embodiment of the present application; and

FIG. 9 is a partially schematic structural diagram of a distal body according to an embodiment of the present application.

## Detailed Description of the Invention

The exemplary implementations of the present invention will be described in more detail below with reference to the accompanying drawings. Although the accompanying drawings show the exemplary implementations of the present application, it should be understood that the present application can be implemented in various forms, and should not be limited to the implementations stated herein. Rather, these implementations are provided for understanding the present application more thoroughly, and can completely transfer the scope of the present invention to those skilled in the art.

It should be noted that the symbol "/" in the present application represents the meaning of "or". For example, "A/B" means "A or B" and "A and/or B" means "A and B, or A or B".

For ease of description, spatial relative relationship terms can be used herein to describe a relationship between an element or feature shown in the figure relative to another element or feature, such as "internal", "external", "inner side", "outer side", "beneath", "below", "on", and "above". This spatial relative relationship term means including different orientations of a device in use or operation, in addition to the orientations depicted in the figures. For example, if a device in a drawing is flipped, an element described as being "below other elements or features" or "under other elements or features" will subsequently be oriented as being "above other elements or features" or "over other elements or features". Therefore, the exemplary term "below" can include two orientations: "above" and "below". The device can be oriented in other ways (rotated 90 degrees or in other directions) and space-correlated descriptors used herein can also be explained correspondingly.

**Test method:**

**1. Radial support force of stent**

In the present invention, a radial support force tester produced by Blockwise Company is used to test a radial support force of an entire valve stent and radial support forces of a proximal body, a distal body, and a stent body of the valve stent through the following method:
A testing method for the radial support force of the entire valve stent includes: after the valve stent (stent) is expanded to its rated diameter D, placing a valve stent portion to be tested into the radial support force tester, simulating an actual stress state of the valve stent within a blood vessel, compressing the entire valve stent to deform under the action of a pressure head, and measuring a pressure intensity exerted on the valve stent when the diameter of the valve stent decreases to 90% of the rated diameter D of the valve stent during radial compression.

A testing method for the radial support forces of the proximal body, the distal body, and the stent body: after expanding the stent to its rated diameter D, since the stent has a horn-shaped end portion, compressing the entire stent to a medium diameter first, then respectively cutting off the stent body, the proximal body, and the distal body, placing a stent portion to be tested into the radial support force tester, simulating an actual stress state of the stent within a blood vessel, compressing the stent portion to deform under the action of a pressure head, and measuring a pressure intensity exerted on the stent when the diameter of the stent portion decreases to 90% of the rated diameter D of the stent during radial compression.

**2. Resistance to parallel plate compression of stent**

In the present invention, the resistance to parallel plate compression of the stent is tested on a microcomputer-controlled universal mechanical testing machine. First, the valve stent is expanded to its rated diameter D. In this case, the length of the valve stent is L. Then, the valve stent is placed between two parallel plates, and a compression force $N_{parallel\ plate}$ applied by the parallel plates to the stent when the valve stent is compressed to 50% of the rated diameter is measured. The resistance to parallel plate compression $F_{parallel\ plate}$ of the stent can be calculated using the following formula, specifically as follows:

$$F_{parallel\ plate} = \frac{N_{parallel\ plate}}{L}$$

It should be noted that for the valve stent with the horn-shaped end portion, the entire valve stent needs to be first compressed to have the same diameter as the stent body and then tested.

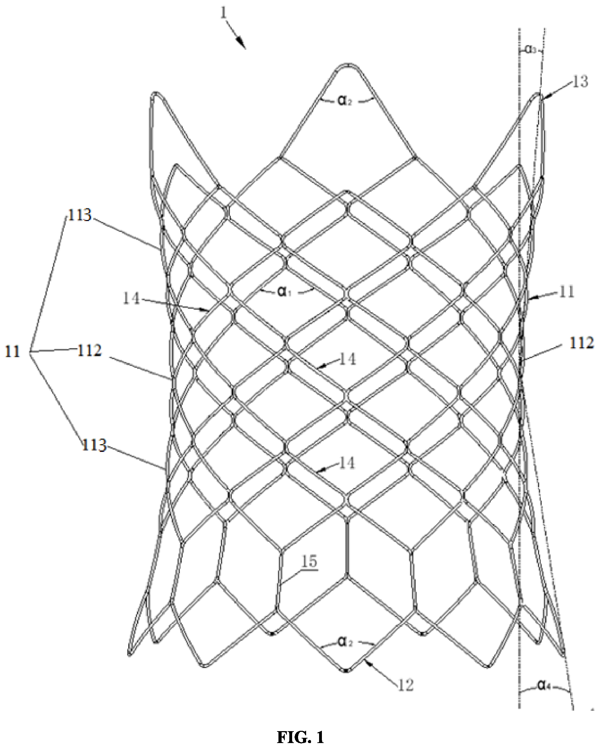

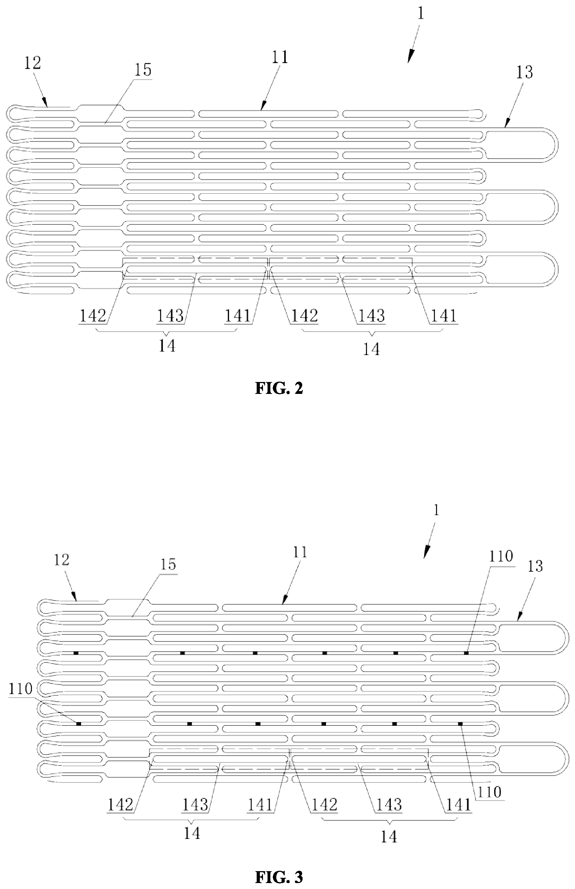

As shown in FIG. 1 and FIG. 2, one embodiment of the present application provides a degradable valve stent 1. The degradable valve stent 1 includes a stent body 11, a proximal body 12, and a distal body 13. The stent body 11 is constructed into a preset shape based on usage requirements and is of a hollow structure to form a channel for allowing blood flow therethrough. The cross section of the stent body 11 perpendicular to an axial direction has a circular contour shape. Certainly, it can be understood that the cross section can also be configured in another shape such as a hexagon or an octagon based on the usage requirements. The proximal body 12 and the distal body 13 are respectively connected to two axial ends of the stent body 11. The stent body 11, the proximal body 12, and the distal body 13 overall form a circumferentially closed ringlike structure, so that the overall structure is stable and has relatively good support performance. The stent body 11, the proximal body 12, and the distal body 13 overall form the circumferentially closed ringlike structure, which means that each of the stent body 11, the proximal body 12, and the distal body 13 is a circumferentially closed ringlike structure, after the stent body 11, the proximal body 12, and the distal body 13 are connected, the whole is still a circumferentially closed ringlike structure. Or, an overall circumferentially closed ringlike structure is formed after the stent body 11, the proximal body 12, and the distal body 13 are connected.

In this embodiment of the present application, the proximal body 12 and the distal body 13 are defined as follows: after the entire degradable valve stent 1 is implanted into a living body, the distance between the valve stent 1 and the heart is used as a reference. One end close to the heart is the proximal body 12, and one end away from the heart is the distal body 13.

According to the degradable valve stent 1 provided in the embodiments of the present application, each of the stent body 11, the proximal body 12, and the distal body 13 is configured to be at least partially degradable, so that the circumferentially closed ringlike structure of the degradable valve stent 1 is deconstructed. That is, each degradable portion fractures upon disintegration, thereby damaging the circumferentially closed structure of the valve stent. Specifically, each of the stent body 11, the proximal body 12, and the distal body 13 is configured to be at least partially degradable. After the degradable portions are degraded, degradable positions fracture. Under the combined effect of the fractured portions, the entire valve stent is deconstructed in a circumferential direction. It is no longer a closed ringlike structure. Thus, the restriction on a radial supporting range is lost, and the restrictive capability no longer exists, so that the valve stent can be expanded. This eliminates the restriction on subsequent reimplantation of a valve stent with a larger radial size and properly meets usage requirements for the degradability of the valve stent and zero restriction on subsequent valve stent reimplantation.

The degradable valve stent 1 provided in this embodiment of the present application is configured into the circumferentially closed ringlike structure by the stent body 11, the proximal body 12, and the distal body 13. Moreover, each of the stent body 11, the proximal body 12, and the distal body 13 is at least partially degradable. After the degradable portions are degraded, the valve stent can be expanded due to the deconstruction of its circumferentially closed ringlike structure. The degradable stent 1 of this structure has the degradability itself, so that it can be degraded and absorbed within preset time, without premature degradation or bringing difficulties and obstructions to subsequent implantation. Furthermore, the entire degradable valve stent 1 has a stable structure and can have appropriate support performance. The strength of the radial supporting meets an application requirement, and the usage reliability is good.

In some possible implementations, the proportion of the total outer surface area of a degradable portion of the degradable valve stent 1 to the total outer surface area of the entire degradable valve stent 1 is greater than or equal to 0.5%. Further, the proportion of the total outer surface area of the degradable portion of the degradable valve stent to the total outer surface area of the entire degradable valve stent is greater than or equal to 5%. Still further, the proportion of the total outer surface area of the degradable portion of the degradable valve stent to the total outer surface area of the entire degradable valve stent is greater than or equal to 99%. To effectively fracture and deconstruct the valve stent after the set degradable portion is degraded, it is necessary to set a sufficiently large region for setting the degradable portion. The "total outer surface area of the entire degradable valve stent 1" refers to the sum of the area of an outer wall and the area of a side wall of the degradable valve stent 1. The side wall refers to a surface connected between the outer wall and an inner wall, while the inner wall refers to a surface facing away from the outer wall, which is also an inner wall surface of an inner cavity of the valve stent. The "total outer surface area of the degradable portion" refers to all areas, belonging to the degradable portion, among the total outer surface area of the entire degradable valve stent 1. In this embodiment of the present application, the proportion of the total outer surface area of the degradable portion to the total outer surface area of the entire degradable valve stent 1 can be optionally set within the above numerical range, for example, greater than or equal to 0.5%, greater than or equal to 5%, or greater than or equal to 99%. Furthermore, this proportion can also be adjusted based on an actual design requirement, provided that the valve stent can be finally reliably deconstructed during the degradation.

The radial support force of the degradable valve stent 1 provided in this embodiment of the present application is [6 kPa, 140 kPa]. Further, the radial support force is [20 kPa, 80 kPa]. Further, the radial support force is [12 kPa, 58 kPa]. Still further, the radial support force is [20 kPa, 58 kPa]. The radial support force refers to a capability of the degradable valve stent 1 to resist a radial compression force. In this way, the degradable valve stent 1 has an appropriate radial support force which can ensure that the degradable valve stent 1 is easily positioned on a valve annulus and a valve is not easily washed away by blood flow, and also avoid phenomenon that an excessive radial support force of the degradable valve stent 1 may damage the valve annulus and an aorta and is not conducive to valve compression for delivery. The degradable valve stent 1 has an appropriate support force and can meet the requirement for a support force.

In some possible embodiments, the radial support force of the degradable valve stent 1 is [10 kPa, 120 kPa], further, [12 kPa, 100 kPa]. The radial support force within the above numerical range meets the current usage requirement for the performance of a magnitude of the radial support force required by the valve stent. That is, the valve stent has the radial support force that meets the usage requirement, which can effectively support a blood vessel and maintain the stability of a supported position and can also facilitate compression and delivery of the valve stent, thus lowering the difficulty in operation and use of the valve stent. Specifically, the performance of the degradable valve stent 1 having different radial support forces may be achieved simultaneously within a single degradable valve stent 1, or may be achieved on different degradable valve stents 1. In this way, corresponding specifications and models can be selected based on actual usage requirements, with good design flexibility to meet usage needs under different conditions.

In some possible implementations, each of the stent body 11, the proximal body 12, and the distal body 13 is configured to be entirely degradable, that is, the entire valve stent can be entirely degraded. In this way, any position of the valve stent can be degraded. During the degradation, the circumferentially closed structure of the valve stent can be damaged by the portions fractured after degradation, instead of waiting for the entire valve stent to be degraded. This increases the possibility of degradation fracture of the valve stent and further improves the reliability that the circumferentially closed structure of the valve stent is damaged after degradation and no longer has the restrictive capability on the radial supporting range.

In this embodiment of the present application, to achieve the degradation of the valve stent within the preset time after the valve stent is implanted into the living body without restricting subsequent reimplantation, the degradable valve stent 1 is configured to be partially or entirely degradable. When the valve stent is partially degradable, the degradable position can deconstruct the entire valve stent without restricting a newly implanted valve. If the degradable portion is disposed on the same straight line in the axial direction, the degradable portion can fracture along this path after degradation, so that the integrity of the valve stent is damaged. The original circumferential restriction performance disappears, and the valve stent can be expanded, for valve stent reimplantation. When the entire valve stent is degradable, that is, any position of the entire degradable valve stent 1 can be degraded, the possibility of degradation fracture of the valve stent is increased. During the degradation, the overall deconstruction of the valve stent can be achieved as long as the circumferentially closed structure of the valve stent can be damaged by the portions fractured after degradation, and the valve stent can be expanded, so that the valve stent no long has the restrictive capability on the radial supporting range, and can meet the usage requirement for reimplantation.

Specifically, the duration during which the degradable valve stent 1 is degraded until the entire valve stent is deconstructed is maintained within a preset time period, and the risk of restenosis in a supported portion due to the disappearance of the support performance caused by premature deconstruction is avoided. The problem of failure of valve stent reimplantation into the same position because of the restriction on reimplantation due to the lack of deconstruction within the preset time period is avoided. The deconstruction time of the degradable valve stent 1 shall not be less than 1 year, and a degradable material shall be completely degraded within 3 to 5 years. When the degradable valve stent 1 is entirely made of a degradable material, the valve stent is a fully degradable valve stent. Within 3 to 5 years, the valve stent is entirely degraded and absorbed by an organism until it disappears completely. In this way, the valve stent provides reliable support for a blood vessel within specified time, and can be completely degraded, without leaving any residue in a body, making it convenient to reimplant a valve stent and also avoiding a long-term potential safety hazard caused by a plurality of valve stents in the body.

The degradable valve stent 1 provided in the present application has a coverage rate of [4%, 18%]. Further, the coverage rate is [4%, 15%]. Further, the coverage rate is [4%, 10%]. Still further, the coverage rate is [4%, 8%). Setting the coverage rate of the degradable valve stent 1 within these numerical ranges can meet the usage requirements. A smaller coverage rate indicates less material usage for the valve stent, which is more conducive to reducing the absorption burden in the body of a patient. Therefore, different degradable valve stents 1 can be disposed separately to match the coverage rates of the above numerical ranges. For example, a coverage rate of one degradable valve stent 1 is 4%, a coverage rate of another degradable valve stent 1 is 8%, a coverage rate of still another degradable valve stent 1 is 12%, and the like. In this way, a degradable valve stent 1 with the most appropriate coverage rate can be selected based on a specific need in practical applications. This improves the accuracy of use of the degradable valve stent 1 and enlarges an applicable range.

It should be noted that the "coverage rate of the degradable valve stent 1" here refers to a coverage rate corresponding to the entire valve stent.

The "coverage rate" in the present application refers to a surface coverage rate of a material after the valve stent is expanded to the rated diameter, i.e. the ratio of the surface area covered by the material of the valve stent to the total outer surface area of a coverage section of the valve stent. The formula is as follows:

surface coverage rate$=A=Sr/Ss \times 100\%$,

where $Sr$ represents an area actually filled/occupied by an outer surface of patterns of the expanded valve stent, and the outer surface area of the valve stent is measured through a computer-aided design (CAD) software;

$Ss$ represents the total outer surface area of the coverage section of the expanded valve stent, $Ss=\pi \times D1 \times L1$;

$D1$ represents the diameter of the expanded valve stent; and

$L1$ represents the length of the expanded valve stent.

The degradable valve stent 1 provided in this embodiment of the present application has a wall thickness of 0.10 mm to 0.40 mm. Further, the wall thickness is 0.15 mm to 0.4 mm. Still further, the wall thickness is 0.18 mm to 0.35 mm. For a patient, as an extracorporeal implant, the valve stent can produce corresponding rejection reactions after implantation, and the rejection reactions will cause a burden on the body of the patient. Therefore, a greater wall thickness of the valve stent and a higher coverage rate can provide stronger support on a blood vessel. However, at the same time, more materials are used, and this is more harmful for the body. Especially when all materials of the valve stent are degradable, more materials are used, more products are produced after the materials are degraded in the body, and a heavier burden of absorbing/clearing the products is imposed on the body of the patient. By controlling the coverage rate and/or the wall thickness of the valve stent, the present application can ensure that the material usage for the degradable valve stent 1 is as minimal as possible to reduce the burden of absorbing/clearing the products in the body of the patient and also provide sufficient support for the valve annulus. Meanwhile, by controlling the wall thickness, the duration during which the valve stent is degraded until the entire valve stent is deconstructed can also be controlled. An appropriate wall thickness is beneficial for the degradation of the valve stent to the deconstruction within the preset time, thus meeting the time requirement within which the valve stent can be supported and can be completely deconstructed.

The degradable valve stent 1 provided in the present application has a resistance to parallel plate compression of not less than 0.05 N/mm or not less than 0.1 N/mm. A specific numerical range can be adjusted based on requirements of different application scenarios. The "resistance to parallel plate compression" evaluates the capability of the degradable valve stent 1 for resisting permanent deformation under a uniformly distributed load along the length of the entire stent. The resistance to parallel plate compression is within this numerical range such that the degradable valve stent 1 has relatively good flattening resistance and can meet the usage requirement of radially undergoing a compression force in a length direction of the entire stent. In this way, this can reduce the damage, such as deformation of a lumen, loss of support on the valve stent, or displacement of a valve leaflet, caused by the degradable valve stent 1 being flattened under radial compression. The degradable valve stent 1 has an appropriate parallel plate compression resistance, which can greatly improve the safety and reliability of use.

In this embodiment of the present application, the degradable valve stent 1 is a valve stent for a pulmonary valve, with a radial support force of [10 kPa, 80 kPa], a coverage rate of [4%, 13%], and a wall thickness of 0.15 mm to 0.35 mm; or the degradable valve stent 1 is a valve stent for an aortic valve, with a radial support force of [30 kPa, 120 kPa], a coverage rate of [5%, 15%], and a wall thickness of 0.18 mm to 0.40 mm; or the degradable valve stent 1 is a valve stent for a mitral valve, with a radial support force of [30 kPa, 100 kPa], a coverage rate of [5%, 13%], and a wall thickness of 0.18 mm to 0.35 mm; or the degradable valve stent 1 is a valve stent for a tricuspid valve, with a radial support force of [30 kPa, 110 kPa], a coverage rate of [5%, 14%], and a wall thickness of 0.18 mm to 0.38 mm.

In some other embodiments, the degradable valve stent is a valve stent for a pulmonary valve, with a radial support force of [6 kPa, 80 kPa], a coverage rate of [4%, 13%], and a wall thickness of 0.15 mm to 0.35 mm; or the degradable valve stent is a valve stent for an aortic valve, with a radial support force of [30 kPa, 140 kPa], a coverage rate of [5%, 18%], and a wall thickness of 0.18 mm to 0.40 mm; or the degradable valve stent is a valve stent for a mitral valve, with a radial support force of [12 kPa, 100 kPa], a coverage rate of [5%, 13%], and a wall thickness of 0.15 mm to 0.35 mm; or the degradable valve stent is a valve stent for a tricuspid valve, with a radial support force of [30 kPa, 110 kPa], a coverage rate of [5%, 14%], and a wall thickness of 0.15 mm to 0.38 mm.

The degradable valve stent 1 provided in the present application has an axial deformation resistance of not less than 0.3 N or not less than 1 N. A specific numerical range can be adjusted based on requirements of different application scenarios. The "axial deformation resistance" refers to a force required by the degradable valve stent 1 in case of irreversible deformation during continuous axial compression. When the deformation resistance is within this numerical range, the degradable valve stent 1 can have relatively good better axial support performance and can meet the usage requirement for axial compression resistance. In this way, this can reduce the damage caused by the irreversible deformation of the degradable valve stent 1 under the axial compression, such as worsening or failure of the support effect or a series of uncontrollable adverse effects including puncturing of a tissue by a protrusion or tip end facing the tissue due to a change in an axial support position. For another example, in a scenario in which an end portion of the degradable valve stent 1 needs to be punctured into a living tissue, if the strength of the axial support force is too small, the end portion is not easily punctured smoothly into the living tissue, or under an axial pressure subsequently, the degradable valve stent 1 may deform and be detached from the living tissue, posing a risk of displacement. The degradable valve stent 1 has an appropriate axial support force, which can greatly improve the safety and reliability of use.

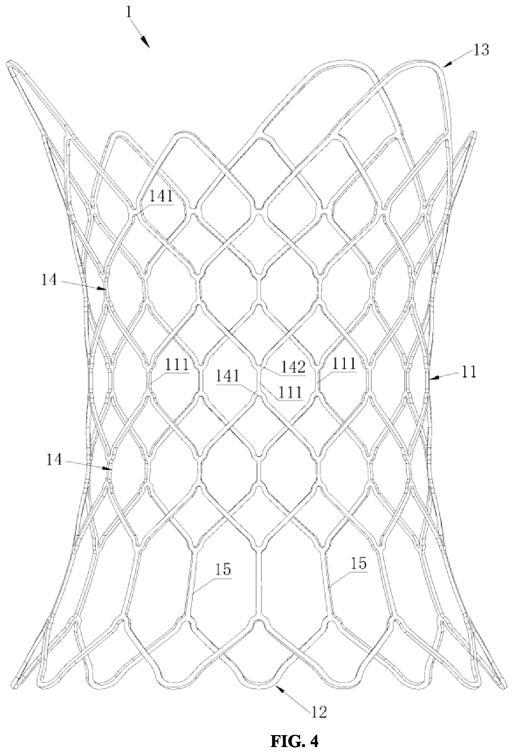

As shown in FIG. 1 and FIG. 4, in this embodiment of the present application, the stent body 11, the proximal body 12, and/or the distal body 13 respectively include a plurality of porous grid structures 14. The porous grid structures 14 are fully closed. By disposing the fully closed porous grid structures 14, connection points can support each other, and the structural strength is high. This not only ensures that the overall support performance of the degradable valve stent 1 can meet the application requirement, but also achieves a uniform expansion effect on the degradable valve stent 1 during expansion under the mutual pulling action of the connection points. Meanwhile, each porous grid structure 14 is hollow, which can also reduce the material usage of the entire degradable valve stent 1 and improve the degradation efficiency of the degradable valve stent 1. Specifically, each formed porous grid structure 14 may be in various shapes, such as a regular polygon or circle (such as a quadrangle in FIG. 1 or a hexagon in FIG. 4), or may be in another irregular shape, with good setting flexibility. The shape of the porous grid structure 14 is not limited here.

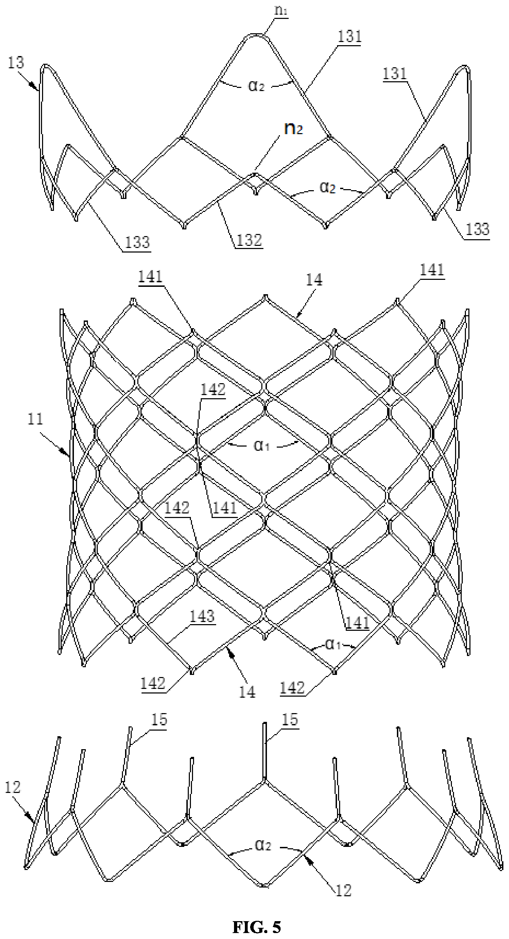

As shown in FIG. 1 and FIG. 5, in one possible implementation, each porous grid structure 14 on the stent body 11 may be configured to include a peak 141 and a trough 142 (defined in a direction from the proximal body 12 to the distal body 13). That is, the porous grid structure 14 is configured as a waveform structure with a regular shape. The porous grid structures 14 are uniformly distributed in the circumferential direction of the stent body 11, and are connected end-to-end and closed in the circumferential direction, to meet at least a usage requirement for radial support performance. The porous grid structures 14 with the regular shapes can be uniformly stressed during expansion, thus achieving uniform expansion. Specifically, an angle $\alpha_1$ between two adjacent support rods 143 that form the peak 141 or the trough 142 on the stent body 11 is 50° to 160°. Preferably, $\alpha_1$ may be 60° to 140° or $\alpha_1$ may be 60° to 120° or $\alpha_1$ may be 70° to 110° or $\alpha_1$ may be 80° to 100°.

Further, as shown in FIG. 4, in one possible implementation, an angle $\alpha_2$ between two adjacent support rods that form a peak $n_1$ or a trough $n_2$ of the proximal body 12 and/or the distal body 13 is 40° to 179°. Preferably, $\alpha_2$ may be 50° to 175°. More preferably, $\alpha_2$ may be 65° to 175°.

Specifically, if an angle formed by adjacent support rods on the same porous grid structure 14 is larger, the waveform structure is less likely to be compressed, and the radial support force is higher. However, if an opening angle is too large, the peaks and the troughs of the stent will be subjected to a higher force, which can easily lead to expandable fracture or long-term fatigue fracture. In the present application, by setting the angle between the two support rods corresponding to each position within the corresponding numerical range, the overall radial support force of the degradable valve stent 1 can be effectively increased, and the overall mechanical performance of the degradable valve stent 1 can meet the application requirement, and the support reliability is good.

According to the degradable valve stent 1 provided in the present application, on the stent body 11, in the circumferential direction of the stent body 11, a total number of 12 to 48 support rods 143 are provided on porous grid structures 14 surrounding the same position. Further, a total number of 12 to 36 support rods are provided. The number of support rods 143 can be adjusted appropriately based on the number of blood vessels near the implantation site of the valve stent and the magnitude of the radial force exerted by surrounding tissues to the valve stent. When there are branch blood vessels near the implantation site of the valve stent, the number of support rods 143 of corresponding porous grid structures 14 can be reduced based on relative positions between the branch blood vessels and the valve stent, so that mesh openings are as large as possible than the ostiums of the branch blood vessels, thereby avoiding shielding of the ostiums of the blood vessels and preventing blockage of blood flows at the branch blood vessels. When a low radial force is exerted from the surrounding tissues to a portion of the valve stent, the number of support rods 143 can also be appropriately reduced, so that the corresponding porous grid structures 14 have sufficient mechanical performance, and the material usage for the valve stent can be reduced, thereby reducing the burden on material absorption and shortening time required for degradation. If there is a branch blood vessel at a distal end of a pulmonary valve, the number of support rods 143 at the distal end of a valve stent for a pulmonary valve can be set to be relatively small, such as 12. Generally, a larger mesh opening is formed in the distal body 13 to avoid shielding of an ostium of a branch blood vessel. In addition, the radial support forces borne by two ends of the pulmonary valve are relatively low, so that both the distal body 13 and the proximal body 12 can be provided with a smaller number of support rods 143.

In this embodiment of the present application, the coverage rate of the distal body 13 is less than or equal to 6%. Optionally, when the structural strength of the distal body 13 meets the support requirement, a smaller coverage rate of the distal body 13 indicates that the distal body 13 uses fewer materials. In this way, fewer products are produced during the degradation. This is more conducive to improving the efficiency of degradation and absorption.

According to the degradable valve stent 1 in this embodiment of the present application, the yield strength of a base material of the degradable valve stent 1 is 350 to 1450 MPa; and/or, the tensile strength of the base material of the degradable valve stent 1 is 400 to 1500 MPa. Specifically, the base material refers to a material that is used in the largest quantity to construct the valve stent. When a portion of the degradable valve stent 1 is made of a degradable material, the base material is a non-degradable material, and the degradable portion mainly plays a role in connection. The yield strength of the base material of the degradable valve stent 1 refers to a yield strength of the non-degradable material. For the partially degradable valve stent 1, a higher strength of the non-degradable material indicates higher fracture resistance. This can lower the risk of fracture caused by long-term presence of the stent in a body. In some other embodiments of the present application, when the degradable valve stent 1 is entirely made of a degradable material, the yield strength of the base material of the degradable valve stent 1 is the yield strength of the degradable material. If the yield strength or tensile strength of the base material is higher, the valve stent uses fewer materials to achieve a corresponding support effect, which is more conducive to reducing the absorption burden in the body of a patient.

According to the degradable valve stent 1 provided in the present application, the material of the degradable portion of the degradable valve stent 1 is at least one of metal or non-metal. The metal at least includes any one of pure iron, iron alloy, pure magnesium, magnesium alloy, pure zinc, or zinc alloy. The non-metal at least includes any one of degradable polyester, degradable polyanhydride, and a bi- or multi-element degradable copolymer formed by copolymerizing monomers corresponding to the degradable polyester and the degradable polyanhydride. The degradable polyester includes at least one of polylactic acid, polyglycolic acid, poly(lactide-co-glycolide), polycaprolactone, polylactide, polyglycolide, polycaprolactide, polyhydroxyalkanoate, polyacrylate, polybutylene succinate, poly($\beta$-hydroxybutyrate), poly(ethylene adipate), and a polyhydroxybutyrate-valerate copolymer. The degradable polyanhydride includes at least one of poly(1,3-bis(p-carboxyphenoxy)propane-sebacic acid), poly(erucic acid dimer-sebacic acid), or poly(fumaric acid-sebacic acid). Or, the non-metal at least includes any one of degradable polyamino acid and a degradable tyrosine-derived polymer. The degradable polyamino acid includes at least one of polyglycine, polyalanine, polyvaline, polyleucine, polyisoleucine, polymethionine, polyproline, polytryptophan, polyserine, polytyrosine, polycysteine, polyphenylalanine, polyasparagine, polyglutamine, polythreonine, polyarginine, polyhistidine, polyselenocysteine, polypyrroline, poly-glutamic acid, poly-aspartic acid, polyornithine, polylysine and derivatives thereof. The degradable tyrosine-derived polymer includes methyl (2R)-2-amino-3-(2-chloro-4-hydroxyphenyl)propanoate, D-tyrosine ethyl ester hydrochloride, methyl (2R)-2-amino-3-(2,6-dichloro-3,4-dimethoxyphenyl)propanoate H-D-Tyr(TBU)-allyl ester HCl, methyl (2R)-2-amino-3-(3-chloro-4,5-dimethoxyphenyl)propanoate, methyl (2R)-2-amino-3-(2-chloro-3-hydroxy-4-methoxyphenyl)propanoate, methyl (2R)-2-amino-3-(4-[(2-chloro-6-fluorophenyl)methoxy]phenyl)propanoate, methyl (2R)-2-amino-3-(2-chloro-3,4-dimethoxyphenyl)propanoate, methyl (2R)-2-amino-3-(3-chloro-5-fluoro-4-hydroxyphenyl)propanoate, 2-(acetylamin)-2(4-[(2-chloro-6-fluorobenzyl)oxy]benzyl)diethyl malonate, methyl (2R)-2-amino-3-(3-chloro-4-methoxyphenyl)propanoate, methyl (2R)-2-amino-3-(3-chloro-4-hydroxy-5-methoxyphenyl)propanoate, methyl (2R)-2-amino-3-(2,6-dichloro-3-hydroxy-4-methoxyphenyl)propanoate, methyl (2R)-2-amino-3-(3-chloro-4-hydroxyphenyl)propanoate, H-DL-tyr-OMe HCl, H-3,5-diiodo-tyr-OME HCl, H-D-3,5-diiodo-tyr-OME HCl, H-D-tyr-OMEHCl, D-tyrosine methyl ester hydrochloride, D-tyrosine-ome HCl, D-tyrosine methyl ester hydrochloride, H-D-tyr-OMe·HCl, D-tyrosine methyl ester HCl, H-D-Tyr-OMe-HCl, (2R)-2-amino-3-(4-hydroxyphenyl)propanoic acid, methyl (2R)-2-amino-3-(4-hydroxyphenyl)hydrochloride, methyl (2R)-2-amino-3-(4-hydroxyphenyl)propanoate hydrochloride, methyl (2R)-2-aminoalkyl-3-(4-hydroxyphenyl)propanoate hydrochloride, 3-chloro-L-tyrosine, 3-nitro-L-tyrosine, 3-nitro-L-tyrosine ethyl ester hydrochloride, DL-m-tyrosine, DL-o-tyrosine, Boc-Tyr(3,5-I2)-OSu, Fmoc-tyr(3-NO$_2$)-OH, $\alpha$-methyl-L-tyrosine, $\alpha$-methyl-D-tyrosine, and $\alpha$-methyl-DL-tyrosine.

As an example in which the material of the degradable portion is metal is used, in some embodiments of the present application, the material of the degradable portion is iron alloy; or the material of the degradable portion is magnesium alloy, zinc alloy, or other alloy; or the material of the degradable material can be a combination of iron alloy and magnesium alloy, or a combination of magnesium alloy and zinc alloy, or a combination of iron alloy and zinc alloy, or a combination of iron alloy, zinc alloy, and magnesium alloy.

It should be noted that "element alloy" in the present application means that the element is contained in the alloy. For example, the iron alloy means that the alloy contains an iron element; the zinc alloy means that the alloy contains a zinc element; and the magnesium alloy means that the alloy contains a magnesium element. Further, "element alloy" means that the mass or volume percentage of the "element" in the alloy is greater than or equal to 0.25%, and 99.75% of the remaining components can be composed of any other metal elements and/or non-metal elements. For example, the iron alloy refers to alloy in which the mass/volume percentage of the iron element is greater than or equal to 0.25%, and so on. Still further, the iron alloy in the present application includes but is not limited to iron-manganese alloy, iron-zinc alloy, iron-magnesium alloy, iron-calcium alloy, iron-zirconium alloy, iron-manganese-carbon alloy, iron-molybdenum alloy, iron-manganese-copper alloy, iron-manganese-silicon-carbon alloy, iron-silicon-manganese alloy, iron-copper alloy, iron-copper-manganese-carbon alloy, iron-gold alloy, iron-silver alloy, iron-manganese-silver alloy, iron-magnesium alloy, iron-hydrogen alloy, iron-phosphorus alloy, iron-sulfur alloy, iron-manganese-carbon alloy, iron-boron alloy, iron-titanium alloy, and iron-titanium-carbon alloy. The zinc alloy includes but is not limited to zinc-aluminum alloy, zinc-silver alloy, zinc-iron alloy, zinc-copper alloy, zinc-iron-calcium alloy, and iron-manganese-copper-carbon alloy. The magnesium alloy includes but is not limited to magnesium-manganese alloy, magnesium-zinc alloy, magnesium-aluminum alloy, and magnesium-iron alloy.

The pure metal in the present application means that the total content of other impurities in the metal is less than or equal to 0.5 wt%. For example, the pure iron means that the total content of other metals and/or non-metals except iron is less than or equal to 0.5 wt%.

In this embodiment of the present application, the degradable valve stent 1 made of the above material can not only achieve degradation within the preset time, but also achieve the purpose of eliminating the restrictive effect through the degradation after implantation, without hindering reimplantation. Moreover, in conjunction with the structural design, the degradable valve stent 1 made of the above material can also have relatively good radial support performance. The radial support force of the valve stent can effectively support a blood vessel and resist an external pressure, so that the valve stent is not likely to collapse and has good support reliability. Meanwhile, the magnitude of the support force of the valve stent cannot cause difficulty in delivery, making it convenient for compression and delivery, with good design ingenuity.

As shown in FIG. 1 and FIG. 5, in some possible embodiments, each porous grid structure 14 is a regular polygon, such as any one of a quadrangle, hexagon, heptagon, and octagon. Or, the porous grid structure 14 can be an irregular polygon, as long as the valve stent can be uniformly expanded during expansion and can provide reliable support, and the irregular polygon may be any shape. Certainly, it can be understood that in some other possible embodiments, the polygonal porous grid structures 14 include regularly polygonal porous grid structures and irregularly polygonal porous grid structures.

In some possible embodiments, the irregular polygonscan be distributed at any position (on the proximal body 12, the distal body 13, and the stent body 11) of the degradable valve stent 1. For example, the on the proximal body 12 are irregular, while the polygons on other portions are regular. Or, the polygons on the distal body 13 are irregular, while the polygons on other portions are regular. Or, the polygons on the stent body 11 are irregular, while the polygons on other portions are regular. In some embodiments, the irregular polygons are non-uniformly distributed; or the irregular polygons are uniformly distributed; or the irregular polygons are distributed at more than one position among the proximal body 12, the distal body 13, and the stent body 11.

According to the degradable valve stent 1 provided in the present application, in a direction from the proximal body 12 to the distal body 13, the peak 141 of an $n^{th}$ porous grid structure 14 on the stent body 11 is connected to the trough 142 of an $(n+1)^{th}$ porous grid structure 14 directly or through a first connection rod 111, where n is greater than or equal to 1. That is, as shown in FIG. 5, in the same direction of the stent body 11, two adjacent porous grid structures 14 may be directly connected. As shown in FIG. 4, two adjacent porous grid structures may be indirectly connected through the first connection rod 111. A specific connection manner can be flexibly selected based on an actual design requirement. The arrangement of the first connection rod 111 increases the distance between two adjacent porous grid structures 14 in a longitudinal direction, thereby increasing the length of the stent body 11. Compared with increasing the length of the stent body 11 by increasing the number of porous grid structures 14 in the length direction, using the first connection rod 111 to increase the length of the stent body can reduce materials used and reduce the coverage rate of the stent body 11. Certainly, it can be understood that an excessive long length of the first connection rod 111 can affect the structural strength of the stent body 11. Therefore, by setting the length of the first connection rod 111 within 0.10 mm to 3.0 mm, the purpose of increasing the length of the stent body 11 can be achieved, and the structural strength of the stent body 11 cannot be affected, thus meeting the need for using degradable valve stents 1 with different lengths.

It should be noted that the first connection rod referred to in the present application can be linear or nonlinear, such as S-shaped, ω-shaped, or Ω-shaped.

As shown in FIG. 5, according to the degradable valve stent 1 provided in the present application, the proximal body 12 and/or the distal body 13 are/is connected to the stent body 11 directly or through a second connection rod 15. That is, the proximal body 12 and/or the distal body 13 can be connected to the stent body 11 directly or through the second connection rod 15. A specific connection manner can be selected according to an actual design requirement. In this embodiment of the present application, as shown in FIG. 5, the second connection rod 15 is disposed between the proximal body 12 and the stent body 11 to connect them. The second connection rod 15 is a straight rod. The disposing of the second connection rod 15 is equivalent to increasing the length of the overall structure of the proximal body 12 and making it easier for the proximal body 12 to bulge and be expanded into a cylindrical shape or an outwardly-flared horn shape, thereby improving the reliability of positioning with an implanted tissue.

Specifically, the proximal body 12 can be configured to be continuously bent into a closed ring with a plurality of ripple structures. When the second connection rod 15 is disposed between the proximal body 12 and the stent body 11 to connect them, one end of the second connection rod 15 can be connected to a peak of the proximal body 12, and another end can be connected to a trough 142, adjacent to the peak, on the stent body 11. Moreover, the number of peaks on the proximal body 12 is equal to the number of adjacent troughs 142 on the stent body 11, so every two adjacent peak and trough 142 are connected through the second connection rod 15, or they are connected at intervals. The peak or the trough is vertical with respect to the valve stent 1. Furthermore, reference in which the proximal body 12 is below and the distal body 13 is above is made.

Further, in some possible embodiments, the length of the second connection rod 15 is 0.8 mm to 4.0 mm; or the length of the second connection rod 15 is 1.5 mm to 3.5 mm. According to the design requirement, the second connection rod 15 can be configured to have different lengths within this range, so that the proximal body 12 can be constructed into different specifications and shapes, to achieve a wide range of applications. Moreover, an excessively long length of the second connection rod 15 can affect the axial support strength and coverage rate of the entire degradable valve stent 1, while an excessively short length of the second connection rod 15 is not conducive for manufacturing. Therefore, setting the length of the second connection rod 15 within the appropriate range can ensure that the entire degradable valve stent 1 has reliable axial support performance and an appropriate coverage rate, and is convenient to produce and process.

As shown in FIG. 1 and FIG. 5, in some possible implementations, the distal body 13 includes first support bodies 131 and second support bodies 132, and one end of each first support body 131 and one end of each second support body 132 are connected to each other, and are connected to the stent body 11. Specifically, each first support body 131 and each second support body 132 are connected to each other in a preset shape to form an "M"-shaped contour having two saw-toothed top ends. Heights of the two top ends can be the same or different. In this structure, the first support body 131 and the second support body 132 support each other, so that the distal body 13 has relatively stable support performance and a relatively high radial support force. Moreover, during expansion, the first support body 131 and the second support body 132 can pull each other, which is conducive to improving the overall expansion uniformity.

As shown in FIG. 5, in some embodiments, each first support body 131 is bent to form a peak $n_1$; each second support body 132 is bent in the same direction to form a peak $n_2$; and the height of the peak $n_2$ of the second support body 132 is less than or equal to 2/3 of the height of the peaks $n_1$ of adjacent first support bodies 131 on each side. The lower height of the peak $n_2$ indicates less obstruction on the second support body. It is more conducive to avoiding shielding of a distal branch blood vessel, thus effectively preventing blockage in the branch blood vessel. Certainly, while meeting the usage requirements, the height relationship between the peak $n_1$ and the peak $n_2$ can also be within other ranges.

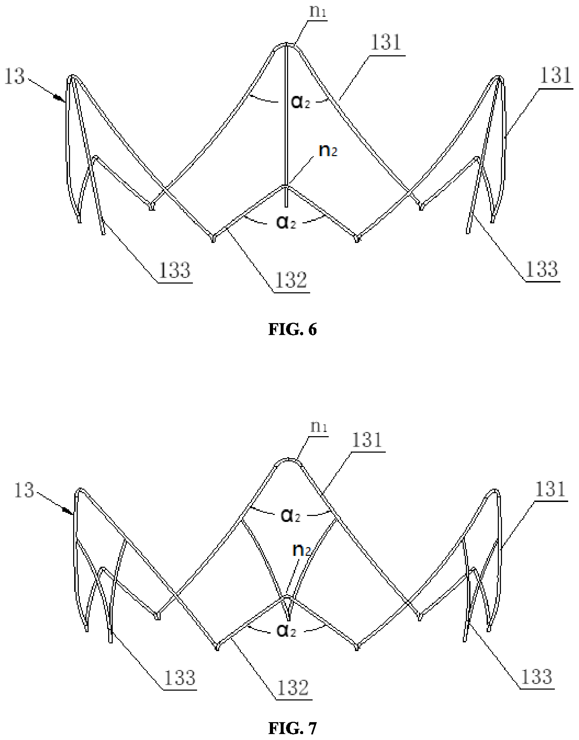

As shown in FIG. 5, in one implementation, the distal body 13 further includes reinforcing members 133. The reinforcing members 133 are arranged between the stent body 11 and the distal body 13 and are at least connected to the first support bodies 131. Two ends of each reinforcing member 133 are respectively connected to two side rods formed by bending of each first support body 131, thereby reinforcing the two side rods and achieving the effect of enhancing the axial support strength and the radial support force of the first support body 131. In another implementation, as shown in FIG. 6, one end of each reinforcing member 133 can be connected to a vertex of the peak $n_1$ formed by the bending of each first support body 131, and another end is connected to any side rod or the stent body 11. This can also achieve the effect of supporting and reinforcing the first support body 131. In this way, the support performance of the distal body 13 can be enhanced by support forces provided by the reinforcing members 133, and the reliability of usage is enhanced. In addition, pulling or limiting of the reinforcing members 133 is also conducive for full expansion of the bent first support bodies 131.

As shown in FIG. 7, in some possible implementations, the reinforcing members 133 are disposed closer to the positions of the vertices of the peaks $n_1$ and are preferably connected within regions between midpoints of the connected side rods and the vertices of the peaks $n_1$. Typically, since the vertices of the peaks $n_1$ are portions to bearing a force first, disposing the reinforcing members 133 closer to the vertices can enhance the strength of the vertices, thus increasing the deformation resistance of the entire distal body 13.

The structural design of the distal body 13 in the degradable valve stent 1 can ensure the uniformity during balloon expansion and after expansion of the distal body 13 and even the entire degradable valve stent 1, and can also ensure that the distal body 13 has a sufficient support force in the axial direction, so that the distal body 13 can be stably fixed in a tissue that is in contact with the distal body 13, to prevent displacement of the valve stent 1 and enhance the effect of fixing the degradable valve stent 1.

In some possible implementations, the entire degradable valve stent 1 is constructed by support rods made of a corresponding material. By using a midpoint of the stent body 11 as a start point, axial cross-sectional areas of the support rods can gradually decrease respectively in directions toward the proximal body 12 and the distal body 13. That is, the axial cross-sectional area at the midpoint of the stent body 11 is maximum, and the axial cross-sectional areas at the vertices of the proximal body 12 and the distal body 13 are minimum. Optionally, a gradual decrease change rate of the axial cross-sectional areas of the support rods is set to 5%. Thus, each support rod itself may not be too thick or too thin, and the proximal body 12 and the distal body 13 use fewer materials for ease of degradation. Particularly, the design in which a portion of the proximal body 12 and/or the distal body 13 that is not adhered to a vascular wall uses fewer materials is conducive to improving the degradation efficiency. Moreover, from the proximal body 12 to the stent body 11 and from the distal body 13 to the stent body 11, the axial cross-sectional areas of the support rods increase progressively, so that the stent body 11 has reliable axial support strength. Thus, the degradable valve stent 1 has good axial deformation resistance and does not easily collapse.

In some possible implementations, the ratio of the maximum diameter at an end portion of the degradable valve stent 1 to a minimum diameter at a middle portion is [1, 1.8]:1. When the ratio of the maximum diameter at the end portion of the valve stent to the minimum diameter at the middle portion is 1:1, the valve stent is cylindrical. When the ratio of the maximum diameter at the end portion of the valve stent to the minimum diameter at the middle portion is greater than 1:1, the valve stent is horn-shaped. Further, the ratio of the maximum diameter at the end portion of the degradable valve stent 1 to the minimum diameter at the middle portion is [1.1, 1.5]:1. Still further, the ratio is [1.1, 1.4]:1. The degradable valve stent 1 exhibits different overall shapes at different ratios. For example, the end portion has different sizes. Thus, a more appropriate valve stent can be correspondingly set based on different application requirements, so that the valve stent can be better adhered to an inner wall of a tissue after implantation, and the applicability is improved.

It should be noted that the "end portion" is a general term for the proximal body 12 and the distal body 13. That is, the end portion includes the proximal body 12 and the distal body 13. The "middle portion" refers to the stent body 11. Therefore, "the ratio of the maximum diameter at the end portion of the degradable valve stent 1 to the minimum diameter at the middle portion is [1.1, 1.8]:1" means that "the ratio of the maximum diameter of the distal body 13 to the minimum diameter of the stent body 11 is [1.1, 1.8]:1, and meanwhile, the ratio of the maximum diameter of the proximal body 12 to the minimum diameter of the stent body 11 is also [1.1, 1.8]:1.

Further, the ratio of the maximum diameter of the proximal body 12 to the minimum diameter of the stent body 11 can be equal to or not equal to the ratio of the maximum diameter of the distal body 13 to the minimum diameter of the stent body 11. That is, the maximum diameter of the proximal body 12 and the maximum diameter of the distal body 13 can be equal or not equal.

Still further, the ratio of the maximum diameter of the proximal body 12 to the minimum diameter of the stent body 11 can be greater than or equal to the ratio of the maximum diameter of the distal body 13 to the minimum diameter of the stent body 11, that is, the maximum diameter of the proximal body 12 is greater than or equal to the maximum diameter of the distal body 13. As shown in FIG. 1, according to the degradable valve stent 1 provided in the present application, the length of the stent body 11 is 15% to 75% of the total length of the valve stent; or the length of the stent body 11 is 20% to 65% of the total length of the valve stent. When it is determined that the total length of the valve stent 1 remains unchanged, stent bodies 11 with different lengths can cause changes in the lengths of the proximal body 12 and the distal body 13. If the stent body 11 is set to be longer, it may result in shorter lengths of the proximal body 12 and/or the distal body 13, so that the angle of inclination of the entire valve stent from the stent body 11 toward the two ends is larger. Conversely, if the stent body 11 is set to be shorter, it may result in longer lengths of the proximal body 12 and/or the distal body 13, so that the angle of inclination of the entire valve stent from the stent body 11 toward the two ends is smaller. Thus, the valve stent can have different forms and a wide range of applications. In this embodiment of the present application, designing the length of the stent body 11 is designed within the above numerical range can meet different design requirements.

As shown in FIG. 1, according to the degradable valve stent 1 provided in the present application, the angle between a connection line from a vertex of the distal body 13 to a midpoint of the stent body 11 and a central axis of the stent body 11 is $\alpha_3$; the angle between a connection line from a vertex of the proximal body 12 to the midpoint of the stent body 11 and the central axis of the stent body 11 is $\alpha_4$; and $\alpha_3$ or $\alpha_4$ is within (0°, 30°], where $\alpha_3$ is equal to $\alpha_4$, or $\alpha_3$ is not equal to $\alpha_4$, or $\alpha_3$ is less than $\alpha_4$.

In some embodiments of the present application, $\alpha_3$ and $\alpha_4$ are both within (0°, 30°]. In some other embodiments of the present application, the angle $\alpha_3$ formed by the distal body 13 is within (0°, 30°], and the angle $\alpha_4$ formed by the proximal body 12 is not within (0°, 30°]. In some other embodiments, the angle $\alpha_3$ formed by the distal body 13 is not within (0°, 30°], and the angle $\alpha_4$ formed by the proximal body 12 is within (0°, 30°].

Further, in some embodiments of the present application, the angle $\alpha_3$ formed by the distal body 13 and the angle $\alpha_4$ formed by the proximal body 12 are consistent, that is, $\alpha_3$ is equal to $\alpha_4$. In some other embodiments of the present application, $\alpha_3$ and $\alpha_4$ are not consistent, that is, $\alpha_3$ is not equal to $\alpha_4$. Still further, the angle $\alpha_3$ formed by the distal body 13 is less than or equal to the angle $\alpha_4$ formed by the proximal body 12.

In this embodiment of the present application, $\alpha_3$ and $\alpha_4$ are respectively set to be different. This can respectively enable the distal body 13 and the proximal body 12 to have different expansion degrees. That is, the distal body 13 and the proximal body 12 can have different sizes, thereby meeting usage requirements for blood vessels with different sizes.

An embodiment of the present application further provides a heart valve including a valve leaflet and the degradable valve stent 1. The valve leaflet is connected to the degradable valve stent 1. The heart valve uses the above degradable valve stent 11. The above degradable valve stent 11 has an appropriate radial support force, can achieve reliable support and uniform expansion, and has good axial deformation resistance and relatively good axial support performance, without causing support collapse. Meanwhile, the degradable valve stent can be degraded to avoid restriction on reimplantation and properly meet usage requirements of the heart valve for effectively supporting a blood vessel, maintaining the stability of a supported position, and reducing the difficulty in a reimplantation operation. Meanwhile, the valve stent is entirely degraded and is absorbed by an organism until it completely disappears, without remaining in a body, thus avoiding a long-term potential safety hazard caused by a plurality of valve stents in the body.

The degradable valve stent 1 provided in the present application includes valve stents for a mitral valve, a tricuspid valve, an aortic valve, and a pulmonary valve.

The degradable valve stent 1 provided in the present application can be directly sutured with the valve leaflet to form an intact valve implant, or can be used as an independent valve stent in which another valve is placed.

After primary corrective surgery is carried out on hypoxemic complex congenital heart diseases (such as Tetralogy of Fallot and pulmonary atresia), residual pulmonary regurgitation (PR) often occurs. It is the most common complication among long-term complications after this type of surgery (particularly using transannular patch repair technique). Severe PR can cause right ventricular outflow tract (RVOT) aneurysmal dilation and dysfunction, pulmonary artery aneurysmal dilation, ventricular systolic asynchrony and arrhythmia, progressive right ventricular dilation/dysfunction, and subsequent right heart failure, ultimately leading to heart failure and death. In recent years, the significance of pulmonary valve reconstruction has attracted increasing attention. Traditional surgical pulmonary valve replacement requires secondary thoracotomy and extracorporeal circulation. Transcatheter pulmonary valve replacement (TPVR) is a minimally invasive surgical technique that has gradually emerged since Bonhoeffer et al. first reported it in 2000. It serves as an alternative to surgical pulmonary valve replacement and improves the long-term prognosis of such patients. TPVR utilizes a pulmonary valve stent for replacement. The pulmonary valve stent generally includes a stent that supports a blood vessel and a valve that replaces an original pulmonary valve. The valve is implanted into a pulmonary valve position through percutaneous intervention surgery through the stent for valve replacement.

Release methods of a transcatheter pulmonary valve can include balloon dilation and self-expanding dilation. Currently, most of valves on the market are self-expanding valves. The valve is placed in a carrier catheter for percutaneous implantation. After the valve reaches a corresponding position, the valve is released and then is fixed to a corresponding annulus by using the structure of the valve stent itself. However, a balloon dilation type stent cannot automatically expand after reaching a corresponding position, and needs to expand through balloon dilation and interactions between the support rods of the valve stent.

Based on the above problems, another embodiment of the present application provides a valve stent for a pulmonary valve. The valve stent 1 can meet requirements of the pulmonary valve, can be expanded uniformly, and has good usage reliability.

As shown in FIG. 1, the valve stent 1 for the pulmonary valve provided in another embodiment of the present application includes a stent body 11, a proximal body 12, and a distal body 13. The stent body 11 is constructed into a hollow tubular structure based on the usage requirement, and can be optionally a structure with a circular axial cross section. A hollow channel inside the stent body 11 is configured for flow of blood therethrough. Certainly, it can be understood that the stent body 11 can also be configured in another shape such as a hexagon or an octagon based on the usage requirement. To achieve a support function, the stent body 11 is configured to include a plurality of porous grid structures 14. After expansion, the porous grid structures 14 can form a support plane with a larger area, thereby achieving the function of supporting an internal tissue of an implanted blood vessel.

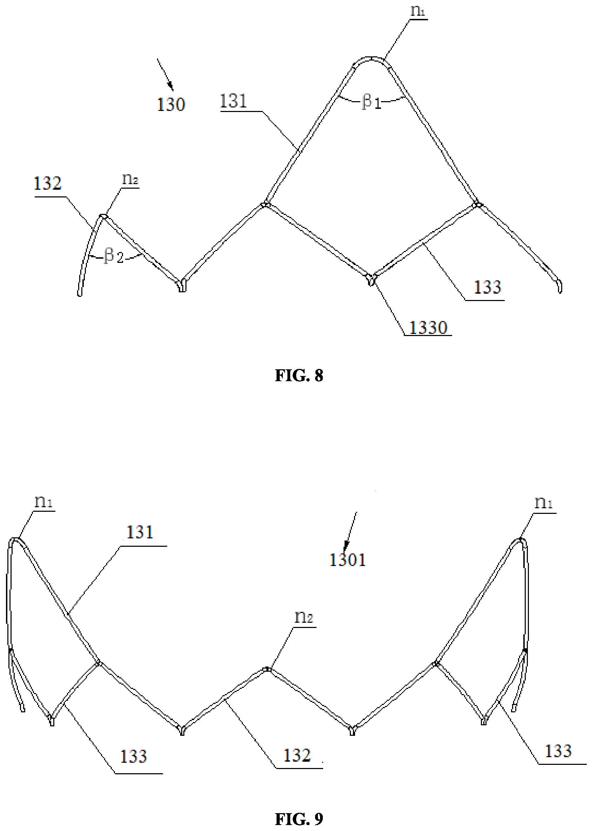

As shown in FIG. 5 and FIG. 8, the distal body 13 is configured to include a plurality of unit bodies 130, each of which has a similarly "M"-shaped contour. The unit bodies 130 are connected end-to-end to form a closed structure, and are connected to the stent body 11 (refer to FIG. 1). In this way, in an expansion process of the entire valve stent 1, since the "M"-shaped unit bodies 130 can pull connection points and constrain themselves when they deform during the expansion, and cooperate with the porous grid structures 14 to play a role in pulling and constraining during the expansion, the entire valve stent 1 can be uniformly expanded and has good consistency in the overall shape. Usage requirements on the valve stent 1 are properly met. The contour of each unit body is "M"-shaped, which is presented by placing the distal body 13 of the valve stent 1 vertically upwards. If the distal body 13 of the valve stent 1 is placed vertically downwards, a "W" shape is presented. That is, referring to different placement manners, shapes intuitively presented by the unit body may be different. For ease of understanding, in this embodiment of the present application, an example in which the distal body 13 of the valve stent 1 is placed vertically upwards and the unit body is presented in an "M" shape is used for description.

Since there are some branch blood vessels near the pulmonary valve, the distal end of the valve stent 1 needs to be designed to be sparse to avoid blockage of the branch blood vessels. In this embodiment of the present application, the number of unit bodies 130 is set to m, where m is a natural number greater than or equal to 2, such as other numbers such as 2, 3, 4, or 5. Different numbers of unit bodies 130 can be combined to achieve different support areas. For example, if the value of m is larger, a larger number of unit bodies 130 are provided, and mesh openings of the valve stent 1 are smaller. Conversely, if the value of m is smaller, a smaller number of unit bodies 130 are provided, and mesh openings of the valve stent 1 are sparse. Therefore, the specific number of unit bodies 130 can be correspondingly set based on different application situations.

It should be noted that the distal body of this embodiment of the present application can be composed of m "M" unit bodies, or can be composed of m "M" unit bodies and in other shapes. Other shapes include but are not limited to "V" shape.

In some possible embodiments, the valve stent 1 for the pulmonary valve of this embodiment of the present application is a balloon dilation type valve stent. A balloon is placed inside the stent. The balloon gradually dilates by inflating the balloon or adding liquid into the balloon, and the valve stent 1 expands outwards by virtue of a force generated by the dilation of the balloon. During the expansion, the connection points can pull each other and provide constraint against deformation, thus achieving uniform force distribution at all positions and synchronous deformation. This achieves a uniform expansion effect and avoids the problem of ineffective support on a blood vessel due to non-uniform expansion in a portion. The expanded valve stent 1 has a uniform shape and good consistency, thus improving the usage reliability of the valve stent 1.

According to the valve stent 1 for the pulmonary valve in this embodiment of the present application, the distal body 13 is configured to include the plurality of unit bodies 130, each of which has an "M"-shaped contour, and the stent body 11 is provided with the plurality of porous grid structures 14. The unit bodies 130 are connected to the stent body 11. In this way, in the expansion process of the entire valve stent 1, since the "M"-shaped unit bodies 130 can pull the connection points and constrain expansion paths, and cooperate with the porous grid structures 14 to play a role in pulling and constraining during the expansion, to achieve a purpose of synchronous expansion, the entire valve stent 1 can uniformly expand and has good consistency in the overall shape.

According to the valve stent 1 in this embodiment of the present application, on the stent body 11, in the circumferential direction of the stent body 11, a total number of 18 to 24 support rods 143 are provided on porous grid structures 14 surrounding the same position.

It should be noted that each unit body 130 of this embodiment of the present application includes a first support body 131 and a second support body 132, and one end of the first support body 131 and one end of the second support body 132 are connected to each other, and are connected to the stent body 11. Specifically, the unit body 130 is constructed by connecting the first support body 131 to the second support body 132 in a preset shape, and has an "M"-shaped contour having two sawtoothed tips. Heights of the two tips can be the same or different. The first support body 131 and the second support body 132 may be the same rod-shaped component that is bent to form the "M"-shaped unit 130 or may be different rod-shaped components that are mutually spliced to form the "M"-shaped unit 130. Through holes are formed inside the two formed tips for blood to pass through. Certainly, it can be understood that the number of first support bodies 131 and/or the number of second support bodies 132 and shapes formed by combining the first support bodies 131 and/or the second support bodies 132, are not limited here while ensuring that each unit body 130 has the "M"-shaped contour and the coverage rate of the constructed valve stent 1 meets a proper usage requirement.

In this embodiment of the present application, one end of each first support body 131 and one end of each second support body 132 that are connected to each other are connected to the peak 141 of the adjacent porous grid structure 14. In this way, during the expansion, the first support body 131 and the second support body 132 can generate a pulling force in an axial direction, thereby achieving mutual pulling between the porous grid structure 14, and the first support body 131, and the second support body 132 that are connected to each other, to guide the expansion deformation. This further improves the expansion uniformity.

As shown in FIG. 5 and FIG. 9, in this embodiment of the present application, a "W"-shaped opening 1301 is formed between two adjacent unit bodies 130, and the ratio of the area of a single opening 1301 to the area of a region formed by combining the distal body 13 with the openings 1301 is greater than or equal to 10%. Specifically, the "W"-shaped opening 1301 formed between two adjacent unit bodies 130 refers to an opening region formed between the first support body 131 of a first unit body 130 and the first support body 131 in an adjacent unit body 130 when the distal body 13 of the valve stent 1 is placed vertically upwards. This region further includes the second support body 132 in the first unit body 130, so the overall contour of the combined opening region is roughly "W"-shaped. It is defined that the distal body 13 and a plurality of openings 1301 are combined to form one region, and the area of each opening 1301 to the area of this region is greater than or equal to 10%. In this way, the overall support strength of the valve stent 1 is within a proper range, and the entire distal body 13 uses fewer materials and has a proper coverage rate.

As shown in FIG. 5 to FIG. 8, the height of a peak $n_2$ of the second support body 132 is less than or equal to 2/3 of a height of a peak $n_1$ of adjacent first support body 131 on each side. Further, the height of the peak $n_2$ of the second support body 132 is less than or equal to 1/2 of the height of the peak $n_1$ of the adjacent first support body 131 on each side. The lower height of the peak $n_2$ indicates a larger "W"-shaped opening 1301 and is more conducive to avoiding shielding of a branch blood vessel of a distal pulmonary artery, thus effectively preventing blockage in the branch blood vessel. Certainly, while meeting the usage requirements, the height relationship between the peak $n_1$ and the peak $n_2$ can also be within other ranges.

As shown in FIG. 8 and FIG. 9, according to the valve stent 1 provided in the above technical solution, the area of the peak $n_2$ in the middle of the "W"-shaped opening 1301 is smaller than or equal to 1/3 of the areas of adjacent peaks $n_1$ on two sides. Further, the area of the peak $n_2$ in the middle of the "W"-shaped opening 1301 is smaller than or equal to 1/4 of the areas of the adjacent peaks $n_1$ on the two sides. The coverage area of the peak $n_1$ cannot be too small because a coverage area that is too small is not easily spread uniformly by the balloon. A coverage area of the peak $n_1$ that is too large easily causes the "W"-shaped opening 1301 to have a small height. This easily blocks the ostium 1301 of the branch blood vessel in the pulmonary artery.

According to the valve stent 1 provided in the above technical solution, the width of the first support body 131 is 1.0 to 3.5 times the width of the second support body 132, and/or the thickness of the first support body 131 is 1.0 to 3.5 times the thickness of the second support body 132. Still further, the width of the first support body 131 is 1.3 to 3 times the width of the second support body 132, and/or the thickness of the first support body 131 is 1.3 to 3 times the thickness of the second support body 132. Still further, the width of the first support body 131 is 1.5 to 2.5 times the width of the second support body 132, and/or the thickness of the first support body 131 is 1.5 to 2.5 times the thickness of the second support body 132. In some embodiments of the present invention, the radial or axial support force of the peak $n_1$ can be increased by increasing the width of the first support body 131. In some other embodiments of the present invention, the radial or axial support force of the peak $n_1$ can be increased by increasing the thickness of the first support body 131. In some other embodiments of the present invention, the radial or axial support force of the peak $n_1$ can be increased by simultaneously increasing the width and thickness of the first support body 131. In this embodiment of the present application, the width of each support rod 143 of the stent body 11 is set to 0.15 mm to 0.5 mm, preferably 0.18 mm to 0.4 mm, and a thickness is set to 0.15 mm to 0.40 mm, preferably 0.18 mm to 0.35 mm.

As shown in the foregoing embodiments, the valve stent for the pulmonary valve of this embodiment of the present application includes reinforcing members. As shown in FIG. 8 and FIG. 9, according to the reinforcing members 133 provided in the above technical solution, while the valve stent 1 meets the usage requirements for the coverage rate, the overall weight, and the like, each first support body 131 can be connected to more than one reinforcing member 133. The desired quantity can be adjusted based on a support need. The shape of each reinforcing member 133 can be set to any shape, such as a bended or folded shape while ensuring a sufficient deformation margin during expansion. When one reinforcing member 133 is connected to each first support body 131, two ends of the reinforcing member 133 are respectively connected to two side rods of the peak $n_1$ formed by the bending of the first support body 131. Furthermore, positions of the reinforcing member 133 connected to the side rods are midpoints of the side rods in a length direction, or are any positions between the midpoints and a vertex of the peak $n_1$. This setting can not only provide a reliable support force for the first support body 131, but also make the reinforcing member 133 closer to the vertex of the peak $n_1$, so that a larger spatial area of a hole formed between the reinforcing member 133 and the stent body 11, which is closer to the branch blood vessel of the pulmonary artery. This reduces obstruction caused by the reinforcing member 133 and facilitates flowing of blood.

As shown in FIG. 5 and FIG. 8, in this embodiment of the present application, each first support body 131 on the distal body 13 is connected to one reinforcing member 133. If three first support bodies 131 are provided, three reinforcing members 133 are also correspondingly provided. Moreover, the two ends of each reinforcing member 133 are respectively connected to the two side rods formed by the bending of the first support body 131. Meanwhile, a bent portion 1330 is formed on the reinforcing member 133, and the bent portion 1330 is configured to be connected to an adjacent peak 141 on the stent body 11. The adjacent peak 141 refers to a peak 141, having the shortest distance from the bent portion 1330 in the porous grid structure 14 on the stent body 11. Connecting the bent portion 1330 to the adjacent peak 141 can further enhance the support effect on the first support body 131. In addition, during the expansion, the reinforcing member 133 and the porous grid structure 14 can pull each other and exert a force to each other. This is more conducive to overall uniform expansion.

According to the valve stent 1 for the pulmonary valve provided in the above technical solution, the percentage of the sum of outer surface areas of the first support bodies 131 to the outer surface area of a cylinder enclosed by the unit bodies 130 is less than or equal to 2.5%; and the percentage of the sum of outer surface areas of the reinforcing members 133 to the outer surface area of the cylinder enclosed by the unit bodies 130 is less than or equal to 2.0%. The outer surface area refers to the total surface area of an object. For example, the outer surface area of each first support body 131 refers to the total area of the surface of the first support body 131. Further, the percentage of the sum of outer surface areas of the first support bodies 131 to the outer surface area of a cylinder enclosed by the unit bodies 130 is less than or equal to 2.3%; and the percentage of the sum of outer surface areas of the reinforcing members 133 to the outer surface area of the cylinder enclosed by the unit bodies 130 is less than or equal to 1.8%. Still further, the percentage of the sum of outer surface areas of the first support bodies 131 to the outer surface area of a cylinder enclosed by the unit bodies 130 is less than or equal to 2.0%; and the percentage of the sum of outer surface areas of the reinforcing members 133 to the outer surface area of the cylinder enclosed by the unit bodies 130 is less than or equal to 1.5%. The ratio of the sum of the outer surface areas of the first support bodies 131 to the outer surface area of the cylinder enclosed by the unit bodies 130 can directly affect the axial and radial support forces of the distal body 13. The distal body 13 of the valve stent 1 cannot be properly fixed in a surrounding tissue, thereby affecting the fixing effect on the valve stent 1. The ratio of the sum of the outer surface areas of the reinforcing members 133 to the outer surface area of the cylinder enclosed by the unit bodies 130 can affect the expansion uniformity of the unit bodies 130 and even the valve stent 1, and finally affect the performance of the entire valve stent 1. Therefore, setting the structures of the first support bodies 131 and the structures of the reinforcing members 133 within proper ranges can enhance the overall reliability of the valve stent 1.

In some possible embodiments, as shown in FIG. 8, an angle $\beta_1$ formed by bending of each first support body 131 is [40°, 110°] and/or an angle $\beta_2$ formed by bending of each second support body 132 is [40°, 179°]. Specifically, the size of the angle $\beta_1$ formed by the bending of the first support body 131 not only affects the support strength, but also affects the shape of a formed tip, thereby affecting the reliability of positioning of the tip puncturing into a tissue. Therefore, optionally, setting the angle $\beta_1$ formed by the bending of the first support body 131 to [40°, 110°] can properly meet the usage requirements. Further, the angle $\beta_1$ formed by the bending of each first support body 131 is [50°, 100°]. Still further, the angle $\beta_1$ formed by the bending of each first support body 131 is [60°, 90°]. The size of the angle $\beta_2$ formed by the bending of the second support body 132 can affect the expansion uniformity of the valve stent 1 under the action of the balloon. If an opening angle is too large, it is not conducive to the expansion of the peaks $n_1$ and the support rods 143 axially connected to the peaks $n_1$, so that the entire valve stent 1 cannot be expanded uniformly and cannot effectively support a tissue. Therefore, optionally, the angle $\beta_2$ formed by the bending of the second support body 132 is set to [40°, 179°]. Further, the angle $\beta_2$ formed by the bending of the second support body 132 is [50°, 170°]. Still further, the angle $\beta_2$ formed by the bending of the second support body 132 is [60°, 160°]. It should be noted that $\beta_1$ and $\beta_2$ can be the same or different.

Further, as shown in FIG. 1, according to the valve stent 1 in this embodiment of the present application, diameters of two ends of the valve stent 1, namely the proximal body 12 and the distal body 13, of the valve stent 1 are slightly greater than the diameter of the stent body 11, thus forming a waist-drum-like structure. The diameter from an end portion to a middle waist 112 gradually decreases, thereby further enhancing the fixing effect of the valve stent 1 and avoiding displacement.

As shown in FIG. 1, according to the valve stent 1 provided in the above technical solution, the ratio of the maximum diameter of the proximal body 12 and/or the distal body 13 of the valve stent 1 to the minimum diameter of the stent body 11 is (1, 1.8]: 1. Further, the ratio of the maximum diameter of the proximal body 12 and/or the distal body 13 to the minimum diameter of the stent body 11 is [1.1, 1.5]: 1. Still further, the ratio of the maximum diameter of the proximal body 12 and/or the distal body 13 to the minimum diameter of the stent body 11 is [1.1, 1.4]: 1.

In some possible implementations, as shown in FIG. 1, according to the valve stent 1 provided in the above technical solution, the stent body 11 has a waist 112 with a consistent diameter, and a connection portion 113 that extends from an edge of the waist 112 toward the proximal body 12 and/or the distal body 13 and has a gradually increasing diameter. The proximal body 12 and the distal body 13 are respectively connected to corresponding connection portions. Since the diameters of the proximal body 12 and the distal body 13 are designed to be different from the diameter of the stent body 11 based on the usage requirements, through the filtering connection of the connection portion 113, the smoothness of the overall structure of the valve stent 1 is ensured.

As shown in FIG. 1, according to the valve stent 1 provided in the above technical solution, the length of the waist 112 of the valve stent 1 is 15% to 60% of the total length of the valve stent 1. Further, the length of the waist 112 of the valve stent 1 is 20% to 40% of the total length of the valve stent 1. When it is determined that the total length of the valve stent 1 remains unchanged, waists 112 with different lengths can cause changes in the lengths of the proximal body 12, the distal body 13, and the connection portion 113. If the waist 112 is set to be longer, the connection portion 113 will be shortened, so that the angle of inclination of the entire valve stent 1 from the waist 112 toward the two ends is larger. Conversely, if the waist 112 is set to be shorter, the connection portion 113 will be lengthened, so that the angle of inclination of the entire valve stent 1 from the waist 112 toward the two ends is smaller. Thus, the valve stent 1 can have different forms and a wide range of applications. In this embodiment of the present application, designing the length of the waist 112 is designed within the above numerical range can meet different design requirements.

As shown in FIG. 1, according to the valve stent 1 provided in the above technical solution, the angle between a connection line from a vertex of the distal body 13 to a midpoint of the waist 112 and a central axis of the stent body 1 is $\alpha_3$; the angle between a connection line from a vertex of the proximal body 12 to the midpoint of the waist 112 and the central axis of the stent body 1 is $\alpha_4$; and $\alpha_3$ or $\alpha_4$ is within (0°, 30°], where $\alpha_3$ is equal to $\alpha_4$, or $\alpha_1$ is not equal to $\alpha_2$, or $\alpha_3$ is less than $\alpha_4$. It can be understood that the angle $\alpha_3$ or $\alpha_4$ between the connection line from the vertex of the proximal body 12/the distal body 13 to the midpoint of the waist 112 and the central axis of the stent body 1 is an angle between a connection line from the vertex of the proximal body 12/the distal body 13 to a midpoint of the stent body 11 and the central axis of the stent body 11.

As mentioned earlier, the proximal body 12 is connected to the peaks 141 of the porous grid structures 14 through a second connection rod 15. Specifically, by disposing the second connection rod 15, the proximal body 12 is connected to opposite peaks 141. The disposing of the second connection rod 15 is equivalent to increasing the length of the overall structure of the proximal body 12 and making it easier for the proximal body 12 to bulge and expand into a cylindrical shape or an outwards flared horn shape, thereby improving the reliability of positioning with an implanted tissue.

Further, the length of the second connection rod 15 is 0.8 mm to 4 mm; or the length of the second connection rod 15 is 1.5 mm to 3.5 mm. According to a design requirement, the second connection rod 15 can be configured to have different lengths within this range, so that the proximal body 12 can be constructed into different specifications and shapes, to achieve a wide range of applications.

According to the valve stent 1 provided in the above technical solution, polygons formed on the distal body 13 and the proximal body 12 are irregular polygons. In some embodiments of the present application, due to the design of a patterned structure of the valve stent 1, polygons formed by a waveform structure of the proximal body 12 or the distal body 13 are naturally irregular polygons when the force of the balloon is consistent. In some other embodiments of the present application, the waveform structure of the proximal body 12 or the distal body 13 itself is designed as a regular polygon, outward forces exerted by the balloon to different positions of the proximal body 12 or the distal body 13 are different, so that the polygons of the proximal body 12 or the distal body 13 are irregular. Even in some embodiments, the polygons formed by the waveform structures of the distal body 13 and the proximal body 12 are non-planar polygons, each of which has a folding curve and forms a three-dimensional structure.

In some possible embodiments, according to the valve stent for the pulmonary valve provided in the present application, the radial support force of the stent body 11 is [6 kPa, 140 kPa], or the radial support force of the stent body 11 is [12 kPa, 100 kPa]. The radial support force of the proximal body 12 is [6 kPa, 120 kPa]. The radial support force of the distal body 13 is [6 kPa, 100 kPa]. The deformation resistance of the distal body 13 is greater than or equal to 1.5 N. The valve stent 1 is configured to have the support force within the above numerical range, so that the magnitude of the radial support force can ensure that the valve stent 1 is easily positioned on a valve annulus, and a valve leaflet is not easily washed away by a blood flow. Meanwhile, this also avoids the phenomenon that the valve annulus and an aorta are damaged because of the generation of an excessively high radial support force of the valve stent 1 is not conducive to compressing a valve for delivery and is not prone to deformation. With the support force performance within the above numerical range, the valve stent 1 properly meets the usage requirements and has good usage reliability.

In the valve stent 1 in this embodiment of the present application, the deformation resistance of the distal body 13 is greater than or equal to 0.5 N (the deformation resistance refers to a force required by the distal body 13 in case of irreversible deformation during continuous compression.). When the deformation resistance is within this numerical range, the distal body 13 has relatively good axial support performance and compressive performance that meets the usage requirements. In this way, this can reduce the damage caused by the irreversible deformation of the distal body 13 under the axial compression, such as worsening or failure of the support effect or a series of uncontrollable adverse effects including puncturing of a tissue by a protrusion or tip end facing the tissue due to a change in an axial support position, and greatly improves the safety and reliability of usage of the valve stent 1.

According to the valve stent 1 provided in the above technical solution, the yield strength of a base material of the valve stent 1 is 350 to 1450 MPa; and/or, the tensile strength of the base material of the valve stent 1 is 400 to 1500 MPa. The base material of the valve stent 1 used in the present application has relatively good yield strength or tensile strength. This can ensure that the valve stent 1 has a smaller diameter and uses fewer materials as much as possible under a corresponding radial support force, so that it is conducive to delivering the valve stent 1 in a body. When the base material of the valve stent 1 is a degradable valve stent 1, the valve stent 1 can reduce the burden of degradation/absorption in the body due to use of fewer materials.

According to the valve stent 1 provided in the above technical solution, the elongation of the base material of the valve stent 1 is greater than or equal to 5%. The base material of the valve stent 1 used in the present application has relatively good elongation, so that the valve stent 1 has relatively good delivery performance in the body. In addition, the valve stent 1 also has better plasticity and does not easily fracture.

The material of the valve stent 1 for the pulmonary valve in this embodiment of the present application may be entirely non-degradable or entirely degradable or partially degradable. When the valve stent is at least partially degradable, the material of a degradable portion of the valve stent may be at least one of metal or nonmetal. The metal at least includes any one of pure iron, iron alloy, pure magnesium, magnesium alloy, pure zinc, or zinc alloy. The non-metal at least includes any one of degradable polyester, degradable polyanhydride, and a bi- or multi-element degradable copolymer formed by copolymerizing monomers corresponding to the degradable polyester and the degradable polyanhydride. The degradable polyester includes at least one of polylactic acid, polyglycolic acid, poly(lactide-co-glycolide), polycaprolactone, polylactide, polyglycolide, polycaprolactide, polyhydroxyalkanoate, polyacrylate, polybutylene succinate, poly($\beta$-hydroxybutyrate), poly(ethylene adipate), and a polyhydroxybutyrate-valerate copolymer. The degradable polyanhydride includes at least one of poly(1,3-bis(p-carboxyphenoxy)propane-sebacic acid), poly(erucic acid dimer-sebacic acid), or poly(fumaric acid-sebacic acid). Or, the non-metal at least includes any one of degradable polyamino acid and a degradable tyrosine-derived polymer. The degradable polyamino acid includes at least one of polyglycine, polyalanine, polyvaline, polyleucine, polyisoleucine, polymethionine, polyproline, polytryptophan, polyserine, polytyrosine, polycysteine, polyphenylalanine, polyasparagine, polyglutamine, polythreonine, polyarginine, polyhistidine, polyselenocysteine, polypyrroline, poly-glutamic acid, poly-aspartic acid, polyornithine, polylysine and derivatives thereof. The degradable tyrosine-derived polymer includes methyl (2R)-2-amino-3-(2-chloro-4-hydroxyphenyl)propanoate, D-tyrosine ethyl ester hydrochloride, methyl (2R)-2-amino-3-(2,6-dichloro-3,4-dimethoxyphenyl)propanoate H-D-Tyr(TBU)-allyl ester HCl, methyl (2R)-2-amino-3-(3-chloro-4,5-dimethoxyphenyl)propanoate, methyl (2R)-2-amino-3-(2-chloro-3-hydroxy-4-methoxyphenyl)propanoate, methyl (2R)-2-amino-3-(4-[(2-chloro-6-fluorophenyl)methoxy]phenyl)propanoate, methyl (2R)-2-amino-3-(2-chloro-3,4-dimethoxyphenyl)propanoate, methyl (2R)-2-amino-3-(3-chloro-5-fluoro-4-hydroxyphenyl)propanoate, 2-(acetylamin)-2(4-[(2-chloro-6-fluorobenzyl)oxy]benzyl)diethyl malonate, methyl (2R)-2-amino-3-(3-chloro-4-methoxyphenyl)propanoate, methyl (2R)-2-amino-3-(3-chloro-4-hydroxy-5-methoxyphenyl)propanoate, methyl (2R)-2-amino-3-(2,6-dichloro-3-hydroxy-4-methoxyphenyl)propanoate, methyl (2R)-2-amino-3-(3-chloro-4-hydroxyphenyl)propanoate, H-DL-tyr-OMe HCl, H-3,5-diiodo-tyr-OME HCl, H-D-3,5-diiodo-tyr-OME HCl, H-D-tyr-OMEHCl, D-tyrosine methyl ester hydrochloride, D-tyrosine-ome HCl, D-tyrosine methyl ester hydrochloride, H-D-tyr-OMe·HCl, D-tyrosine methyl ester HCl, H-D-Tyr-OMe-HCl, (2R)-2-amino-3-(4-hydroxyphenyl)propanoic acid, methyl (2R)-2-amino-3-(4-hydroxyphenyl)hydrochloride, methyl (2R)-2-amino-3-(4-hydroxyphenyl)propanoate hydrochloride, methyl (2R)-2-aminoalkyl-3-(4-hydroxyphenyl)propanoate hydrochloride, 3-chloro-L-tyrosine, 3-nitro-L-tyrosine, 3-nitro-L-tyrosine ethyl ester hydrochloride, DL-m-tyrosine, DL-o-tyrosine, Boc-Tyr(3,5-I2)-OSu, Fmoc-tyr(3-NO$_2$)-OH, $\alpha$-methyl-L-tyrosine, $\alpha$-methyl-D-tyrosine, and $\alpha$-methyl-DL-tyrosine.

It should be noted that the pure metal in this embodiment of the present application means that the total content of other impurities in the metal is less than or equal to 0.5 wt%. For example, the pure iron means that a total content of other metals and/or non-metals except iron is less than or equal to 0.5 wt%.

The valve stent 1 provided in the present invention can be directly sutured with the valve leaflet to form an intact valve implant, or can be used as an independent valve stent 1 in which another valve leaflet is placed.

An embodiment of the present application further provides a pulmonary valve including a valve leaflet and the above valve stent 1. The valve leaflet is connected to the valve stent 1. The pulmonary valve uses the above valve stent 1. The valve stent 1 can not only expand uniformly, but also have a proper radial support force. The magnitude of the support force ensures that the valve stent 1 is easily positioned on a valve annulus and prevents the valve leaflet from being easily washed away by a blood flow. This greatly improves the reliability of usage of the pulmonary valve.

It should be noted that in some embodiments, the degradable valve stent of the above embodiments can be applied to a valve stent for a pulmonary valve. It can be understood that the structures, parameters, and effects described for the above degradable valve stent 1 can be combined with the valve stent for the pulmonary valve. That is, structures that are not specifically described in structures of the valve stent for the pulmonary valve can be replaced with the structures of the degradable valve stent of the above embodiments.

All data in the present application refer to corresponding values of a middle portion of the valve stent 1 at a nominal diameter. The "nominal diameter" refers to the diameter of the middle portion of the valve stent 1 when the valve stent 1 completely expands under a nominal pressure, and a nominal diameter refers to the diameter of the valve stent 1 when the valve stent completely expands under the nominal pressure. The nominal pressure refers to the pressure required for the complete expansion of the valve stent 1 in clinical practice. The complete expansion refers to a state in which the valve stent 1 expands to match the diameter of a lumen to which the valve stent is applied.

An interval value range of $X_1$-$X_2$ in the present application represents that a value can be $X_1$, $X_2$, or any value within an interval between $X_1$ and $X_2$. That is, the interval is a closed interval. For example, the present application describes that "a wall thickness of a stent is 0.04 mm to 0.5 mm", which means the wall thickness of the stent can be 0.04 mm, 0.5 mm, or any value between 0.04 mm and 0.5 mm.

For ease of understanding of the present application, the design points of the present application will be described in conjunction with some specific embodiments below. It can be understood that the relevant embodiments are only some examples of the solutions of the present application and do not constitute limitations on the range of applications.

Embodiment 1

This embodiment provides a degradable valve stent 1 which is made of nitrided iron having a tensile strength of 850 MPa and a yield strength of 750 MPa. The degradable valve stent 1 includes a stent body 11, a proximal body 12, and a distal body 13. The stent body 11 is formed by connecting a plurality of porous grid structures. Each porous grid structure in the same circumferential direction is formed by combining a total of 36 support rods. Each support rod has a wall thickness of 0.23 mm and a coverage rate of 6.6%. A coverage rate of the distal body 13 is 2.5%. The valve stent 1 is designed to be fully closed, with an angle $\alpha_1$ of 50° and an angle $\alpha_2$ of 40°. The distal body 13 is directly connected to the stent body 11, and the proximal body 12 is connected to the stent body 11 through a second connection rod 15 with a length of 4.0 mm. The length of the stent body 11 accounts for 15% of the total length of the valve stent. Two ends of the valve stent 1 upwarp. The diameter of the stent body 11 is 20 mm. The ratio of the maximum diameter of the proximal body 12 to the maximum diameter of the stent body 11 and the ratio of the maximum diameter of the distal body 13 to the maximum diameter of the stent body 11 are both 1.2. The angle between a connection line from a vertex of the distal body 13 to a midpoint of the stent body 11 and a central axis of the stent body 11 is $\alpha_3$, and the angle between a connection line from a vertex of the proximal body 12 to the midpoint of the stent body 11 and the central axis of the stent body 11 is $\alpha_4$. Both $\alpha_3$ and $\alpha_4$ are 8°. The distal body 13 includes first support bodies 131 and second support bodies 132. Each first support body 131 is bent to form a peak $n_1$, and each second support body 132 is bent to form a peak $n_2$. The height of the peak $n_2$ is 1/2 of the height of the peak $n_1$. In addition, reinforcing members 133 are arranged on two side rods formed by the bending of each first support body 131, and the reinforcing members 133 are respectively connected to midpoints of the two side rods in a length direction.

The valve stent has a radial support force of 18 kPa, an axial deformation resistance of 3.5 N, and a resistance to parallel plate compression of 0.2 N/mm. The valve stent can be implanted into the pulmonary valve of a dog through an interventional method. This stent can be properly fixed in a lumen, without displacement, and can support the lumen and not affect a blood flow in a distal branch blood vessel. At the 12th month, a second valve stent is implanted in situ, and the implantation process is smooth. The expansion of the valve stent is not restricted, and the blood vessel can still be expanded to an expected diameter. Later, the valve stent is withdrawn. It can be observed that the valve stent implanted for the first time has been significantly degraded. The valve stent implanted for the second time will be completely degraded in approximately three years.

Embodiment 2

This embodiment provides a degradable valve stent 1 which is made of nitrided iron having a tensile strength of 950 MPa and a yield strength of 900 MPa. The degradable valve stent 1 includes a stent body 11, a proximal body 12, and a distal body 13. The stent body 11 is formed by connecting a plurality of porous grid structures. Each porous grid structure in the same circumferential direction is formed by combining a total of 36 support rods. Each support rod has a wall thickness of 0.18 mm and a coverage rate of 7.1%. The coverage rate of the distal body 13 is 2.8%. The valve stent 1 is designed to be fully closed, with an angle $\alpha_1$ of 60° and an angle $\alpha_2$ of 50°. The distal body 13 is directly connected to the stent body 11, and the proximal body 12 is connected to the stent body 11 through a second connection rod 15 with a length of 0.8mm. The length of the stent body 11 accounts for 75% of the total length of the valve stent. The distal body 13 upwarps into a flared shape. The diameter of the stent body 11 is 20 mm. The the ratio of a maximum diameter of the distal body 13 to a maximum diameter of the stent body 11 is 1.1. The angle between a connection line from a vertex of the distal body 13 to a midpoint of the stent body 11 and a central axis of the stent body 11 is $\alpha_3$ which is 5°, and the angle between a connection line from a vertex of the proximal body 12 to the midpoint of the stent body 11 and the central axis of the stent body 11 is $\alpha_4$ which is 0°. The distal body 13 includes first support bodies 131 and second support bodies 132. Each first support body 131 is bent to form a peak $n_1$, and each second support body 132 is bent to form a peak $n_2$. The height of the peak $n_2$ is 1/2 of the height of the peak $n_1$. In addition, reinforcing members 133 are arranged on two side rods formed by the bending of each first support body 131, and the reinforcing members 133 are respectively connected to midpoints of the two side rods in a longitudinal direction.

The valve stent has a radial support force of 30 kPa, an axial deformation resistance of 2.1 N, and a resistance to parallel plate compression of 0.16 N/mm. The valve stent can be implanted into the aortic valve of a dog through an interventional method. This stent can be properly fixed in a lumen, without displacement, and can support the lumen and not affect a blood flow in a distal branch blood vessel. At the 12th month, a second valve stent is implanted in situ, and the implantation process is smooth. The expansion of the valve stent is not restricted by the original stent, and the blood vessel can still be expanded to an expected diameter. Later, the valve stent is withdrawn. It can be observed that the valve stent implanted for the first time has been significantly degraded. The valve stent implanted for the second time will be completely degraded in approximately three years.

Embodiment 3

This embodiment provides a degradable valve stent 1. It is specifically a zinc-based absorbable valve stent for a mitral valve, which is made of zinc alloy having a tensile strength of 480 MPa and a yield strength of 380 MPa. The degradable valve stent 1 includes a stent body 11, a proximal body 12, and a distal body 13. The stent body 11 is formed by connecting a plurality of porous grid structures. Each porous grid structure in the same circumferential direction is formed by combining a total of 36 support rods. Each support rod has a wall thickness of 0.15 mm and a coverage rate of 9.2%. The coverage rate of the distal body 13 is 3.8%. The valve stent 1 is designed to be fully closed, with an angle $\alpha_1$ of 120° and an angle $\alpha_2$ of 65°. The distal body 13 is directly connected to the stent body 11, and the proximal body 12 is connected to the stent body 11 through a second connection rod 15 with a length of 0.8 mm. The length of the stent body 11 accounts for 65% of the total length of the valve stent. The distal body 13 upwarps into a flared shape. The diameter of the stent body 11 is 16 mm. The the ratio of a maximum diameter of the distal body 13 to a maximum diameter of the stent body 11 is 1.3. The angle between a connection line from a vertex of the distal body 13 to a midpoint of the stent body 11 and a central axis of the stent body 11 is $\alpha_3$ which is 13°, and the angle between a connection line from a vertex of the proximal body 12 to the midpoint of the stent body 11 and the central axis of the stent body 11 is $\alpha_4$ which is 0°. The distal body 13 includes first support bodies 131 and second support bodies 132. Each first support body 131 is bent to form a peak $n_1$, and each second support body 132 is bent to form a peak $n_2$. The height of the peak $n_2$ is 1/2 of the height of the peak $n_1$. In addition, reinforcing members 133 are arranged on two side rods formed by the bending of each first support body 131, and the reinforcing members 133 are respectively connected to midpoints of the two side rods in a length direction.

The valve stent has a radial support force of 12 kPa, an axial deformation resistance of 1.2 N, and a resistance to parallel plate compression of 0.10 N/mm. The valve stent can be implanted into the mitral valve of a dog through an interventional method. The valve stent can be properly fixed in a lumen, without displacement, and can support the lumen and not affect blood flow in a distal branch blood vessel. At the 12th month, a second valve stent is implanted in situ, and the implantation process is smooth. The expansion of the valve stent is not restricted by the original stent, and the blood vessel can still be expanded to an expected diameter. Later, the valve stent is withdrawn. It can be observed that the valve stent implanted for the first time has been significantly degraded. The valve stent implanted for the second time will be completely degraded in approximately three years.

Embodiment 4

This embodiment provides a degradable valve stent 1. It is specifically an iron-based absorbable valve stent for a tricuspid valve, which is made of nitrided iron having a tensile strength of 900 MPa and a yield strength of 830 MPa. The degradable valve stent 1 includes a stent body 11, a proximal body 12, and a distal body 13. The stent body 11 is formed by connecting a plurality of porous grid structures. Each porous grid structure in the same circumferential direction is formed by combining a total of 36 support rods. Each support rod has a wall thickness of 0.15mm and a coverage rate of 8.0%. A coverage rate of the distal body 13 is 2.4%. The stent is designed to be fully closed, with an angle $\alpha_1$ of 160° and an angle $\alpha_2$ of 175°. The distal body 13 is directly connected to the stent body 11, and the proximal body 12 is connected to the stent body 11 through a second connection rod 15 with a length of 0.8 mm. The length of the stent body 11 accounts for 75% of the total length of the valve stent. The distal body 13 upwarps into a flared shape. The diameter of the stent body 11 is 18 mm. The the ratio of a maximum diameter of the distal body 13 to a maximum diameter of the stent body 11 is 1.8. The angle between a connection line from a vertex of the distal body 13 to a midpoint of the stent body 11 and a central axis of the stent body 11 is $\alpha_3$ which is 30°, and the angle between a connection line from a vertex of the proximal body 12 to the midpoint of the stent body 11 and the central axis of the stent body 11 is $\alpha_4$ which is 0°. The distal body 13 includes first support bodies 131 and second support bodies 132. Each first support body 131 is bent to form a peak $n_1$, and each second support body 132 is bent to form a peak $n_2$. The height of the peak $n_2$ is 1/2 of a height of the peak $n_1$. In addition, reinforcing members 133 are arranged on two side rods formed by the bending of each first support body 131, and the reinforcing members 133 are respectively connected to midpoints of the two side rods in a length direction.

The valve stent has a radial support force of 40 kPa, an axial deformation resistance of 1.8 N, and a resistance to parallel plate compression of 0.18 N/mm. The valve stent can be implanted into the tricuspid valve of a dog through an interventional method. The valve stent can be properly fixed in a lumen, without displacement, and can support the lumen and not affect a blood flow in a distal branch blood vessel. At the 12th month, a second stent is implanted in situ, and the implantation process is smooth. The expansion of the valve stent is not restricted by the original stent, and the blood vessel can still be expanded to an expected diameter. Later, the valve stent is withdrawn. It can be observed that the valve stent implanted for the first time has been significantly degraded. The stent implanted for the second time will be completely degraded in approximately three years.

Embodiment 5

This embodiment provides a degradable valve stent 1. It is specifically an iron-based absorbable valve stent for a pulmonary valve, which is made of nitrided iron having a tensile strength of 950 MPa and a yield strength of 900 MPa. The degradable valve stent 1 includes a stent body 11, a proximal body 12, and a distal body 13. The stent body 11 is formed by connecting a plurality of porous grid structures. Each porous grid structure in the same circumferential direction is formed by combining a total of 36 support rods. Each support rod has a wall thickness of 0.35 mm and a coverage rate of 13%. The coverage rate of the distal body 13 is 5.5%. The stent is designed to be fully closed, with an angle $\alpha_1$ of 100° and an angle $\alpha_2$ of 90°. The distal body 13 is directly connected to the stent body 11, and the proximal body 12 is connected to the stent body 11 through a second connection rod 15 with a length of 1.5 mm. The length of the stent body 11 accounts for 20% of the total length of the valve stent. Two ends of the valve stent 1 upwarp. The diameter of the stent body 11 is 16 mm. The ratio of the maximum diameter of the proximal body 12 to the maximum diameter of the stent body 11 and the ratio of the maximum diameter of the distal body 13 to the maximum diameter of the stent body 11 are both 1.3. The angle between a connection line from a vertex of the distal body 13 to a midpoint of the stent body 11 and a central axis of the stent body 11 is $\alpha_3$, and the angle between a connection line from a vertex of the proximal body 12 to the midpoint of the stent body 11 and the central axis of the stent body 11 is $\alpha_4$. Both $\alpha_3$ and $\alpha_4$ are 9°. The distal body 13 includes first support bodies 131 and second support bodies 132. Each first support body 131 is bent to form a peak $n_1$, and each second support body 132 is bent to form a peak $n_2$. The height of the peak $n_2$ is 1/2 of the height of the peak $n_1$. In addition, reinforcing members 133 are arranged on two side rods formed by the bending of each first support body 131, and the reinforcing members 133 are respectively connected to midpoints of the two side rods in a length direction.

The valve stent 1 has a radial support force of 80 kPa, an axial deformation of 5.0 N, and a resistance to parallel plate compression of 0.5 N/mm. The valve stent 1 can be implanted into the pulmonary valve of a dog through an interventional method. The valve stent can be properly fixed in a lumen, without displacement, and can support the lumen and not affect a blood flow in a distal branch blood vessel. At the 12th month, a second valve stent is implanted in situ, and the implantation process is smooth. The expansion of the valve stent is not restricted by the original stent, and the blood vessel can still be expanded to an expected diameter. Later, the valve stent is withdrawn. It can be observed that the valve stent implanted for the first time has been significantly degraded. The valve stent implanted for the second time will be completely degraded in approximately four years.

Embodiment 6

This embodiment provides a degradable valve stent 1. It is specifically an iron-based absorbable valve stent for a pulmonary valve, which is made of pure iron having a tensile strength of 650 MPa and a yield strength of 480 MPa. The degradable valve stent 1 includes a stent body 11, a proximal body 12, and a distal body 13. The stent body 11 is formed by connecting a plurality of porous grid structures. Each porous grid structure in the same circumferential direction is formed by combining a total of 36 support rods. Each support rod has a wall thickness of 0.25 mm and a coverage rate of 5.0%. The coverage rate of the distal body 13 is 2.1%. The stent is designed to be fully closed, with an angle $\alpha_1$ of 90° and an angle $\alpha_2$ of 75°. The distal body 13 is directly connected to the stent body 11, and the proximal body 12 is connected to the stent body 11 through a second connection rod 15 with a length of 1.5 mm. The length of the stent body 11 accounts for 20% of the total length of the valve stent. Two ends of the valve stent 1 upwarp. The diameter of the stent body 11 is 24 mm. The ratio of a maximum diameter of the proximal body 12 to the maximum diameter of the stent body 11 and the ratio of the maximum diameter of the distal body 13 to the maximum diameter of the stent body 11 are both 1.3. The angle between a connection line from a vertex of the distal body 13 to a midpoint of the stent body 11 and a central axis of the stent body 11 is $\alpha_3$, and the angle between a connection line from a vertex of the proximal body 12 to the midpoint of the stent body 11 and the central axis of the stent body 11 is $\alpha_4$. Both $\alpha_3$ and

$\alpha_4$ are 9°. The distal body 13 includes first support bodies 131 and second support bodies 132. Each first support body 131 is bent to form a peak $n_1$, and each second support body 132 is bent to form a peak $n_2$. The height of the peak $n_2$ is 1/3 of a height of the peak $n_1$. In addition, reinforcing members 133 are arranged on two side rods formed by the bending of each first support body 131, and the reinforcing members 133 are respectively connected between midpoints of the two side rods in a length direction and the peak $n_1$. The support reliability is good.

The valve stent has a radial support force of 6 kPa, an axial deformation resistance of 2.5 N, and a resistance to parallel plate compression of 0.12 N/mm. The valve stent can be implanted into the pulmonary valve of a dog through an interventional method. The valve stent can be properly fixed in a lumen, without displacement, and can support the lumen and not affect a blood flow in a distal branch blood vessel. At the 12th month, a second valve stent is implanted in situ, and the implantation process is smooth. The expansion of the valve stent is not restricted by the original valve stent, and the blood vessel can still be expanded to an expected diameter. Later, the valve stent is withdrawn. It can be observed that the valve stent implanted for the first time has been significantly degraded. The valve stent implanted for the second time will be completely degraded in approximately three and a half years.

Embodiment 7

This embodiment provides a degradable valve stent 1. It is specifically an iron-based absorbable valve stent for an aortic valve, which is made of iron-manganese alloy having a tensile strength of 1500 MPa and a yield strength of 1450 MPa. The degradable valve stent 1 includes a stent body 11, a proximal body 12, and a distal body 13. The stent body 11 is formed by connecting a plurality of porous grid structures. Each porous grid structure in the same circumferential direction is formed by combining a total of 48 support rods. Each support rod has a wall thickness of 0.40mm and a coverage rate of 18%. The coverage rate of the distal body 13 is 6%. The stent is designed to be fully closed, with an angle $\alpha_1$ of 140° and an angle $\alpha_2$ of 90°. The distal body 13 is directly connected to the stent body 11, and the proximal body 12 is connected to the stent body 11 through a second connection rod 15 with a length of 0.8 mm. The length of the stent body 11 accounts for 50% of the total length of the valve stent. The distal body 13 upwarps into a flared shape. The diameter of the stent body 11 is 16 mm. The the ratio of a maximum diameter of the distal body 13 to a maximum diameter of the stent body 11 is 1.5. The angle between a connection line from a vertex of the distal body 13 to a midpoint of the stent body 11 and a central axis of the stent body 11 is $\alpha_3$ which is 15°, and the angle between a connection line from a vertex of the proximal body 12 to the midpoint of a middle portion of the stent body 11 and the central axis of the stent body 11 is $\alpha_4$

which is 0°. The distal body 13 includes first support bodies 131 and second support bodies 132. Each first support body 131 is bent to form a peak $n_1$, and each second support body 132 is bent to form a peak $n_2$. The height of the peak $n_2$ is 1/2 of the height of the peak $n_1$. In addition, reinforcing members 133 are arranged on two side rods formed by the bending of each first support body 131, and the reinforcing members 133 are respectively connected to midpoints of the two side rods in a longitudinal direction.

The valve stent has a radial support force of 140 kPa, an axial deformation resistance of 6.5 N, and a resistance to parallel plate compression of 0.8 N/mm. The valve stent can be implanted into the aortic valve of a dog through an interventional method. The valve stent can be properly fixed in a lumen, without displacement, and can support the lumen and not affect the blood flow in a distal branch blood vessel. At the 12th month, a second valve stent is implanted in situ, and the implantation process is smooth. The expansion of the valve stent is not restricted by the original valve stent, and the blood vessel can still be expanded to an expected diameter. Later, the valve stent is withdrawn. It can be observed that the valve stent implanted for the first time has been significantly degraded. The valve stent implanted for the second time will be completely degraded in approximately five years.

Embodiment 8

This embodiment provides a degradable valve stent 1. It is specifically an iron-based absorbable valve stent for an aortic valve, which is made of nitrided iron having a tensile strength of 950 MPa and a yield strength of 900 MPa. The degradable valve stent 1 includes a stent body 11, a proximal body 12, and a distal body 13. The stent body 11 is formed by connecting a plurality of porous grid structures. Each porous grid structure in the same circumferential direction is formed by combining a total of 36 support rods. Each support rod has a wall thickness of 0.35 mm and a coverage rate of 15%. The coverage rate of the distal body 13 is 5.8%. The stent is designed to be fully closed, with an angle $\alpha_1$ of 120° and an angle $\alpha_2$ of 90°. The distal body 13 is directly connected to the stent body 11, and the proximal body 12 is connected to the stent body 11 through a second connection rod 15 with a length of 0.8 mm. The length of the stent body 11 accounts for 50% of the total length of the valve stent. The distal body 13 upwarps into a flared shape. The diameter of the stent body 11 is 16 mm. The the ratio of a maximum diameter of the distal body 13 to a maximum diameter of the stent body 11 is 1.5. The angle between a connection line from a vertex of the distal body 13 to a midpoint of the stent body 11 and a central axis of the stent body 11 is $\alpha_3$ which is 15°, and the angle between a connection line from a vertex of the proximal body 12 to the midpoint of the stent body 11 and the central axis of the stent body 11 is $\alpha_4$ which is 0°. The distal body 13 includes first support bodies 131 and second support bodies 132. Each first support body 131 is bent to form a peak $n_1$, and each second support body 132 is bent to form a peak $n_2$. The height of the peak $n_2$ is 1/2 of the height of the peak $n_1$. In addition, reinforcing members 133 are arranged on two side rods formed by the bending of each first support body 131, and the reinforcing members 133 are respectively connected to midpoints of the two side rods in a longitudinal direction.

The valve stent has a radial support force of 120 kPa, an axial deformation resistance of 5.2 N, and a resistance to parallel plate compression of 0.6 N/mm. The valve stent can be implanted into the aortic valve of a dog through an interventional method. The valve stent can be properly fixed in a lumen, without displacement, and can support the lumen and not affect a blood flow in a distal branch blood vessel. At the 12th month, a second valve stent is implanted in situ, and the implantation process is smooth. The expansion of the valve stent is not restricted by the original stent, and the blood vessel can still be expanded to an expected diameter. Later, the valve stent is withdrawn. It can be observed that the valve stent implanted for the first time has been significantly degraded. The valve stent implanted for the second time will be completely degraded in approximately four and a half years.

Embodiment 9

This embodiment provides a degradable valve stent 1. It is specifically an iron-based absorbable valve stent for a mitral valve, which is made of nitrided iron having a tensile strength of 800 MPa and a yield strength of 720 MPa. The degradable valve stent 1 includes a stent body 11, a proximal body 12, and a distal body 13. The stent body 11 is formed by connecting a plurality of porous grid structures. Each porous grid structure in the same circumferential direction is formed by combining a total of 36 support rods. Each support rod has a wall thickness of 0.35 mm and a coverage rate of 13%. The coverage rate of the distal body 13 is 5.5%. The stent is designed to be fully closed, with an angle $\alpha_1$ of 110° and an angle $\alpha_2$ of 90°. The distal body 13 is directly connected to the stent body 11, and the proximal body 12 is connected to the stent body 11 through a second connection rod 15 with a length of 0.8 mm. The length of the stent body 11 accounts for 35% of the total length of the valve stent. The distal body 13 upwarps into a flared shape. The diameter of the stent body 11 is 16 mm. The the ratio of a maximum diameter of the distal body 13 to a maximum diameter of the stent body 11 is 1.3. The angle between a connection line from a vertex of the distal body 13 to a midpoint of the stent body 11 and a central axis of the stent body 11 is $\alpha_3$ which is 9°, and the angle between a connection line from a vertex of the proximal body 12 to the midpoint of the stent body 11 and the central axis of the stent body 11 is $\alpha_4$ which is 0°. The distal body 13 includes first support bodies 131 and second support bodies 132. Each first support body 131 is bent to form a peak $n_1$, and each second support body 132 is bent to form a peak $n_2$. The height of the peak $n_2$ is 1/2 of the height of the peak $n_1$. In addition, reinforcing members 133 are arranged on two side rods formed by the bending of each first support body 131, and the reinforcing members 133 are respectively connected to midpoints of the two side rods in a longitudinal direction.

The valve stent 1 has a radial support force of 100 kPa, an axial deformation resistance of 5.1 N, and a resistance to parallel plate compression of 0.5 N/mm. The valve stent 1 can be implanted into the mitral valve of a dog through an interventional method. The valve stent can be properly fixed in a lumen, without displacement, and can support the lumen and not affect a blood flow in a distal branch blood vessel. At the 12th month, a second valve stent is implanted in situ, and the implantation process is smooth. The expansion of the valve stent is not restricted by the original valve stent, and the blood vessel can still be expanded to an expected diameter. Later, the valve stent is withdrawn. It can be observed that the valve stent implanted for the first time has been significantly degraded. The valve stent implanted for the second time will be completely degraded in approximately four years.

Embodiment 10

This embodiment provides a degradable valve stent 1. It is specifically an iron-based absorbable valve stent for a tricuspid valve, which is made of nitrided iron having a tensile strength of 900 MPa and a yield strength of 830 MPa. The degradable valve stent 1 includes a stent body 11, a proximal body 12, and a distal body 13. The stent body 11 is formed by connecting a plurality of porous grid structures. Each porous grid structure in the same circumferential direction is formed by combining a total of 36 support rods. Each support rod has a wall thickness of 0.38 mm and a coverage rate of 14%. The coverage rate of the distal body 13 is 5.2%. The stent is designed to be fully closed, with an angle $\alpha_1$ of 70° and an angle $\alpha_2$ of 175°. The distal body 13 is directly connected to the stent body 11, and the proximal body 12 is connected to the stent body 11 through a second connection rod 15 with a length of 0.8 mm. The length of the stent body 11 accounts for 75% of the total length of the valve stent. The distal body 13 upwarps into a flared shape. The diameter of the stent body 11 is 16 mm. The the ratio of the maximum diameter of the distal body 13 to a maximum diameter of the stent body 11 is 1.8. The angle between a connection line from a vertex of the distal body 13 to a midpoint of the stent body 11 and a central axis of the stent body 11 is $\alpha_3$ which is 30°, and the angle between a connection line from a vertex of the proximal body 12 to the midpoint of the stent body 11 and the central axis of the stent body 11 is $\alpha_4$ which is 30°. The distal body 13 includes first support bodies 131 and second support bodies 132. Each first support body 131 is bent to form

a peak $n_1$, and each second support body 132 is bent to form a peak $n_2$. The height of the peak $n_2$ is 1/2 of the height of the peak $n_1$. In addition, reinforcing members 133 are arranged on two side rods formed by the bending of each first support body 131, and the reinforcing members 133 are respectively connected to midpoints of the two side rods in a length direction.

The valve stent has a radial support force of 110 kPa, an axial deformation resistance of 5.8 N, and a resistance to parallel plate compression of 0.5 N/mm. The valve stent can be implanted into the tricuspid valve of a dog through an interventional method. The valve stent can be properly fixed in a lumen, without displacement, and can support the lumen and not affect a blood flow in a distal branch blood vessel. At the 12th month, a second valve stent is implanted in situ, and the implantation process is smooth. The expansion of the valve stent is not restricted by the original valve stent, and the blood vessel can still be expanded to an expected diameter. Later, the valve stent is withdrawn. It can be observed that the valve stent implanted for the first time has been significantly degraded. The valve stent implanted for the second time will be completely degraded in approximately four and a half years.

Embodiment 11

This embodiment provides a degradable valve stent 1. It is specifically an iron-based absorbable valve stent for a pulmonary valve, which is made of nitrided iron having a tensile strength of 750 MPa and a yield strength of 850 MPa. The degradable valve stent 1 includes a stent body 11, a proximal body 12, and a distal body 13. The stent body 11 is formed by connecting a plurality of porous grid structures. Each porous grid structure in the same circumferential direction is formed by combining a total of 12 support rods and has a coverage rate of 4.0%. By using a midpoint of the stent body 11 as a start point, respectively in directions toward the proximal body 12 and the distal body 13, the wall thickness of a support rod at the midpoint is 0.25 mm, and wall thicknesses of support rods at two ends gradually decrease to 0.15 mm. The angle between a connection line from a vertex of the proximal body 12 or the distal body 13 to the midpoint of the stent body 11 and an axis of a stent rod is 0.4°. The coverage rate of the distal body 13 is 1.5%. The stent is designed to be fully closed, with an angle $\alpha_1$ of 90° and an angle $\alpha_2$ of 75°. The distal body 13 is directly connected to the stent body 11, and the proximal body 12 is connected to the stent body 11 through a second connection rod 15 with a length of 3.5 mm. The length of the stent body 11 accounts for 20% of the total length of the valve stent. Two ends of the valve stent 1 upwarp. The diameter of the stent body 11 is 16 mm. The ratio of the maximum diameter of the proximal body 12 to the maximum diameter of the stent body 11 and the ratio of a maximum diameter of the distal body 13 to the maximum diameter of the stent body 11 are both 1.2. The angle between a connection line from a vertex of the distal body 13 to a midpoint of the stent body 11 and a central axis of the stent body 11 is $\alpha_3$, and the angle between a connection line from a vertex of the proximal body 12 to the midpoint of the stent body 11 and the central axis of the stent body 11 is $\alpha_4$. Both $\alpha_3$ and $\alpha_4$ are 8°. The distal body 13 includes first support bodies 131 and second support bodies 132. Each first support body 131 is bent to form a peak $n_1$, and each second support body 132 is bent to form a peak $n_2$. The height of the peak $n_2$ is 1/2 of a height of the peak $n_1$. In addition, reinforcing members 133 are arranged on two side rods formed by the bending of each first support body 131, and the reinforcing members 133 are respectively connected to midpoints of the two side rods in a length direction.

The valve stent has a radial support force of 12 kPa, an axial deformation of 2.7 N, and a resistance to parallel plate compression of 0.22 N/mm. The valve stent can be implanted into the pulmonary valve of a dog through an interventional method. The valve stent can be well fixed in a lumen, without displacement, and can support the lumen and not affect blood flow in a distal branch blood vessel. At the 12th month, a second valve stent is implanted in situ, and the implantation process is smooth. The expansion of the valve stent is not restricted by the original stent, and the blood vessel can still be expanded to an expected diameter. Later, the valve stent is withdrawn. It can be observed that the valve stent implanted for the first time has been significantly degraded. The valve stent implanted for the second time will be completely degraded in approximately four years.

Embodiment 12

This embodiment provides a degradable valve stent 1. It is specifically a magnesium-based absorbable valve stent for a pulmonary valve, which has a tensile strength of 400 MPa and a yield strength of 350 MPa. The degradable valve stent 1 includes a stent body 11, a proximal body 12, and a distal body 13. The stent body 11 and the proximal body 12 are made of magnesium alloy, and the distal body 13 is made of a cobalt-chromium alloy material and a magnesium alloy material. The magnesium alloy only constitutes part of support rods, mainly plays a role in connection, and accounts for 5% of the distal body. The remaining portion is made of the cobalt-chromium alloy material and accounts for 10% of the distal body, namely, 90.5% of the stent can be degraded. The stent body 11 is formed by connecting a plurality of porous grid structures. Each porous grid structure in the same circumferential direction is formed by combining a total of 36 support rods. Each support rod has a wall thickness of 0.23 mm and a coverage rate of 6.2%. The coverage rate of the distal body 13 is 2.2%. The stent is designed to be fully closed, with an angle $\alpha_1$ of 160° and an angle $\alpha_2$ of 179°. The distal body 13 is directly connected to the stent body 11, and the proximal body 12 is connected to the stent body 11 through a second connection rod 15 with a length of 3.5 mm. The length of the stent body 11 accounts for 20% of the total length of the valve stent. Two ends of the valve stent 1 upwarp. The diameter of the stent body 11 is 20mm. The ratio of the maximum diameter of the proximal body 12 to the maximum diameter of the stent body 11 and the ratio of a maximum diameter of the distal body 13 to the maximum diameter of the stent body 11 are both 1.2. The angle between a connection line from a vertex of the distal body 13 to a midpoint of the stent body 11 and a central axis of the stent body 11 is $\alpha_3$, and the angle between a connection line from a vertex of the proximal body 12 to the midpoint of the stent body 11 and the central axis of the stent body 11 is $\alpha_4$. Both $\alpha_3$ and $\alpha_4$ are 8°. The distal body 13 includes first support bodies 131 and second support bodies 132. Each first support body 131 is bent to form a peak $n_1$, and each second support body 132 is bent to form a peak $n_2$. The height of the peak $n_2$ is 1/2 of the height of the peak $n_1$. In addition, reinforcing members 133 are arranged on two side rods formed by the bending of each first support body 131, and the reinforcing members 133 are respectively connected to midpoints of the two side rods in a length direction.

The valve stent has a radial support force of 10 kPa, an axial deformation resistance of 1.8 N, and a resistance to parallel plate compression of 0.1 N/mm. The valve stent can be implanted into the pulmonary valve of a dog through an interventional method. The valve stent can be properly fixed in a lumen, without displacement, and can support the lumen and not affect blood flow in a distal branch blood vessel. A non-degradable distal end portion of the valve stent is located at a pulmonary artery ostium, significantly upwarps, and can be adhered to a vascular wall. At the 12th month, a second valve stent is implanted in situ, and the implantation process is smooth. The expansion of the valve stent is not restricted by the original valve stent, and the blood vessel can still be expanded to an expected diameter. Later, the valve stent is withdrawn. It can be observed that the stent body 11 and the proximal body 12 in the valve stent 1 implanted for the first time have been significantly degraded and the distal body 13 has been completely deconstructed.

Embodiment 13

This embodiment provides a degradable valve stent 1. It is specifically a valve stent for a pulmonary valve. A main material is non-degradable cobalt-chromium alloy having a tensile strength of 1500 MPa and a yield strength of 1450 MPa. The degradable valve stent 1 is formed by combining a plurality of support rods and includes a stent body 11, a proximal body 12, and a distal body 13. The stent body 11 is formed by connecting a plurality of porous grid structures. Each porous grid structure in the same circumferential direction is formed by combining a total of 36 support rods. Each support rod has a wall thickness of 0.23 mm and a coverage rate of 6.6%. As shown in FIG. 3, part of the support rods in the valve stent 1 are not continuous and are connected through connection rods 110 made of a degradable polylactic acid material. The remaining portion is made of a non-degradable cobalt-chromium alloy material. The ratio of areas of all the connection rods 110 to the area of the valve stent 1 is 0.5%. That is, 0.5% of the valve stent 1 is degradable. The coverage rate of the distal body 13 is 2.5%. The stent is designed to be fully closed, with an angle $\alpha_1$ of 90° and an angle $\alpha_2$ of 75°. The distal body 13 is directly connected to the stent body 11, and the proximal body 12 is connected to the stent body 11 through a second connection rod 15 with a length of 3.5 mm. The length of the stent body 11 accounts for 20% of the total length of the valve stent. Two ends of the valve stent 1 upwarp. The diameter of the stent body 11 is 20 mm. The ratio of the maximum diameter of the proximal body 12 to the maximum diameter of the stent body 11 and the ratio of the maximum diameter of the distal body 13 to the maximum diameter of the stent body 11 are both 1.2. The angle between a connection line from a vertex of the distal body 13 to a midpoint of the stent body 11 and a central axis of the stent body 11 is $\alpha_3$, and the angle between a connection line from a vertex of the proximal body 12 to the midpoint of the stent body 11 and the central axis of the stent body 11 is $\alpha_4$. Both $\alpha_3$ and $\alpha_4$ are 8°. The distal body 13 includes first support bodies 131 and second support bodies 132. Each first support body 131 is bent to form a peak $n_1$, and each second support body 132 is bent to form a peak $n_2$. The height of the peak $n_2$ is 1/2 of the height of the peak $n_1$. In addition, reinforcing members 133 are arranged on two side rods formed by the bending of each first support body 131, and the reinforcing members 133 are respectively connected to midpoints of the two side rods in a longitudinal direction.

The valve stent has a radial support force of 45 kPa, an axial deformation resistance of 4.2 N, and a resistance to parallel plate compression of 0.18 N/mm. The valve stent can be implanted into the pulmonary valve of a dog through an interventional method. This stent can be properly fixed in a lumen, without displacement, and can support the lumen and not affect a blood flow in a distal branch blood vessel. At the 12th month, a second valve stent is implanted in situ, and the implantation process is smooth. The expansion of the valve stent is not restricted by the original stent, and the blood vessel can still be expanded to an expected diameter. Later, the valve stent is withdrawn. It can be observed that the connection rods 110 in the valve stent 1 implanted for the first time have been degraded. The stent has been deconstructed.

Embodiment 14

This embodiment provides a degradable valve stent 1. It is specifically an iron-based absorbable valve stent for a pulmonary valve, which is made of nitrided iron having a tensile strength of 950 MPa and a yield strength of 900 MPa. The degradable valve stent 1 includes a stent body 11, a proximal body 12, and a distal body 13. The stent body 11 is formed by connecting a plurality of porous grid structures. Each porous grid structure in the same circumferential direction is formed by combining a total of 36 support rods. Each support rod has a wall thickness of 0.15 mm and a coverage rate of 9%. The coverage rate of the distal body 13 is 5%. The stent is designed to be fully closed, with an angle $\alpha_1$ of 110° and an angle $\alpha_2$ of 90°. The distal body 13 is directly connected to the stent body 11, and the proximal body 12 is connected to the stent body 11 through a second connection rod 15 with a length of 1.5 mm. The length of the stent body 11 accounts for 20% of the total length of the valve stent. Two ends of the valve stent 1 upwarp. The diameter of the stent body 11 is 16 mm. The ratio of the maximum diameter of the proximal body 12 to the maximum diameter of the stent body 11 and the ratio of a maximum diameter of the distal body 13 to the maximum diameter of the stent body 11 are both 1.3. The angle between a connection line from a vertex of the distal body 13 to a midpoint of the stent body 11 and a central axis of the stent body 11 is $\alpha_3$, and the angle between a connection line from a vertex of the proximal body 12 to the midpoint of the stent body 11 and the central axis of the stent body 11 is $\alpha_4$. Both $\alpha_3$ and $\alpha_4$ are 8°. The distal body 13 includes first support bodies 131 and second support bodies 132. Each first support body 131 is bent to form a peak $n_1$, and each second support body 132 is bent to form a peak $n_2$. The height of the peak $n_2$ is 1/2 of the height of the peak $n_1$. In addition, reinforcing members 133 are arranged on two side rods formed by the bending of each first support body 131, and the reinforcing members 133 are respectively connected to midpoints of the two side rods in a longitudinal direction.

The valve stent 1 has a radial support force of 65 kPa, an axial deformation of 4.0 N, and a resistance to parallel plate compression of 0.4 N/mm. The valve stent 1 can be implanted into the pulmonary valve of a dog through an interventional method. The valve stent can be properly fixed in a lumen, without displacement, and can support the lumen and not affect blood flow in a distal branch blood vessel. At the 12th month, a second valve stent is implanted in situ, and the implantation process is smooth. The expansion of the valve stent is not restricted by the original stent, and the blood vessel can still be expanded to an expected diameter. Later, the valve stent is withdrawn. It can be observed that the valve stent implanted for the first time has been significantly degraded.

Embodiment 15

This embodiment provides a degradable valve stent 1 which is made of nitrided iron having a tensile strength of 950 MPa and a yield strength of 900 MPa. The degradable valve stent 1 includes a stent body 11, a proximal body 12, and a distal body 13. The stent body 11 is formed by connecting a plurality of porous grid structures. Each porous grid structure in the same circumferential direction is formed by combining a total of 36 support rods. Each support rod has a wall thickness of 0.3mm and a coverage rate of 5%. The coverage rate of the distal body 13 is 2.1%. The valve stent 1 is designed to be fully closed, with an angle $\alpha_1$ of 70° and an angle $\alpha_2$ of 50°. The distal body 13 is directly connected to the stent body 11, and the proximal body 12 is connected to the stent body 11 through a second connection rod 15 with a length of 0.8mm. The length of the stent body 11 accounts for 75% of the total length of the valve stent. The distal body 13 upwarps into a flared shape. The diameter of the stent body 11 is 20 mm. The the ratio of the maximum diameter of the distal body 13 to a maximum diameter of the stent body 11 is 1.1. The angle between a connection line from a vertex of the distal body 13 to a midpoint of the stent body 11 and a central axis of the stent body 11 is $\alpha_3$ which is 10°, and the angle between a connection line from a vertex of the proximal body 12 to the midpoint of the stent body 11 and the central axis of the stent body 11 is $\alpha_4$ which is 0°. The distal body 13 includes first support bodies 131 and second support bodies 132. Each first support body 131 is bent to form a peak $n_1$, and each second support body 132 is bent to form a peak $n_2$. The height of the peak $n_2$ is 1/2 of the height of the peak $n_1$. In addition, reinforcing members 133 are arranged on two side rods formed by the bending of each first support body 131, and the reinforcing members 133 are respectively connected to midpoints of the two side rods in a longitudinal direction.

The valve stent has a radial support force of 50 kPa, an axial deformation resistance of 3 N, and a resistance to parallel plate compression of 0.23 N/mm. The valve stent can be implanted into the aortic valve of a dog through an interventional method. The stent can be properly fixed in a lumen, without displacement, and can support the lumen and not affect blood flow in a distal branch blood vessel. At the 12th month, a second valve stent is implanted in situ, and the implantation process is smooth. The expansion of the valve stent is not restricted by the original stent, and the blood vessel can still be expanded to an expected diameter. Later, the valve stent is withdrawn. It can be observed that the valve stent implanted for the first time has been significantly degraded.

Embodiment 16

This embodiment provides a degradable valve stent 1. It is specifically an iron-based absorbable valve stent for a mitral valve, which is made of nitrided iron having a tensile strength of 900 MPa and a yield strength of 800 MPa. The degradable valve stent 1 includes a stent body 11, a proximal body 12, and a distal body 13. The stent body 11 is formed by connecting a plurality of porous grid structures. Each porous grid structure in the same circumferential direction is formed by combining a total of 36 support rods. Each support rod has a wall thickness of 0.18mm and a coverage rate of 5%. The coverage rate of the distal body 13 is 2%. The valve stent 1 is designed to be fully closed, with an angle $\alpha_1$ of 100° and an angle $\alpha_2$ of 90°. The distal body 13 is directly connected to the stent body 11, and the proximal body 12 is connected to the stent body 11 through a second connection rod 15 with a length of 0.8 mm. The length of the stent body 11 accounts for 50% of the total length of the valve stent. The distal body 13 upwarps into a flared shape. The diameter of the stent body 11 is 16 mm. The the ratio of the maximum diameter of the distal body 13 to a maximum diameter of the stent body 11 is 1.3. The angle between a connection line from a vertex of the distal body 13 to a midpoint of the stent body 11 and a central axis of the stent body 11 is $\alpha_3$ which is 8°, and the angle between a connection line from a vertex of the proximal body 12 to the midpoint of the stent body 11 and the central axis of the stent body 11 is $\alpha_4$ which is 8°. The distal body 13 includes first support bodies 131 and second support bodies 132. Each first support body 131 is bent to form a peak $n_1$, and each second support body 132 is bent to form a peak $n_2$. The height of the peak $n_2$ is 1/2 of the height of the peak $n_1$. In addition, reinforcing members 133 are arranged on two side rods formed by the bending of each first support body 131, and the reinforcing members 133 are respectively connected to midpoints of the two side rods in a length direction.

The valve stent has a radial support force of 30 kPa, an axial deformation resistance of 1.8 N, and a resistance to parallel plate compression of 0.18 N/mm. The valve stent can be implanted into the mitral valve of a dog through an interventional method. The valve stent can be properly fixed in a lumen, without displacement, and can support the lumen and not affect blood flow in a distal branch blood vessel. At the 12th month, a second valve stent is implanted in situ, and the implantation process is smooth. The expansion of the valve stent is not restricted by the original stent, and the blood vessel can still be expanded to an expected diameter. Later, the valve stent is withdrawn. It can be observed that the valve stent implanted for the first time has been significantly degraded.

Embodiment 17

This embodiment provides a degradable valve stent 1. It is specifically an iron-based absorbable valve stent for a tricuspid valve, which is made of nitrided iron having a tensile strength of 900 MPa and a yield strength of 830 MPa. The degradable valve stent 1 includes a stent body 11, a proximal body 12, and a distal body 13. The stent body 11 is formed by connecting a plurality of porous grid structures. Each porous grid structure in the same circumferential direction is formed by combining the total of 36 support rods. Each support rod has a wall thickness of 0.18mm and a coverage rate of 5%. The coverage rate of the distal body 13 is 2%. The stent is designed to be fully closed, with an angle $\alpha_1$ of 90° and an angle $\alpha_2$ of 85°. The distal body 13 is directly connected to the stent body 11, and the proximal body 12 is connected to the stent body 11 through a second connection rod 15 with a length of 0.8 mm. The length of the stent body 11 accounts for 60% of the total length of the valve stent. The distal body 13 upwarps into a flared shape. The diameter of the stent body 11 is 16 mm. The the ratio of the maximum diameter of the distal body 13 to a maximum diameter of the stent body 11 is 1.6. The angle between a connection line from a vertex of the distal body 13 to a midpoint of the stent body 11 and a central axis of the stent body 11 is $\alpha_3$ which is 30°, and the angle between a connection line from a vertex of the proximal body 12 to the midpoint of the stent body 11 and the central axis of the stent body 11 is $\alpha_4$ which is 30°. The distal body 13 includes first support bodies 131 and second support bodies 132. Each first support body 131 is bent to form a peak $n_1$, and each second support body 132 is bent to form a peak $n_2$. The height of the peak $n_2$ is 1/2 of the height of the peak $n_1$. In addition, reinforcing members 133 are arranged on two side rods formed by the bending of each first support body 131, and the reinforcing members 133 are respectively connected to midpoints of the two side rods in a length direction.

The valve stent has a radial support force of 30 kPa, an axial deformation resistance of 2 N, and a resistance to parallel plate compression of 0.2 N/mm. The valve stent can be implanted into the tricuspid valve of a dog through an interventional method. The valve stent can be properly fixed in a lumen, without displacement, and can support the lumen and not affect blood flow in a distal branch blood vessel. At the 12th month, a second valve stent is implanted in situ, and the implantation process is smooth. The expansion of the valve stent is not restricted by the original valve stent, and the blood vessel can still be expanded to an expected diameter. Later, the valve stent is withdrawn. It can be observed that the valve stent implanted for the first time has been significantly degraded.

Embodiment 18

This embodiment provides an iron-based absorbable valve stent 1 for a pulmonary valve, which is made of nitrided iron having a tensile strength of 850 MPa and a yield strength of 750 MPa. The valve stent 1 includes a stent body 11, a proximal body 12, and a distal body 13. The stent body 11 is formed by connecting a plurality of porous grid structures. Each porous grid structure in the same circumferential direction is formed by combining 18 support rods. Each support rod has a wall thickness of 0.23 mm and a rod width of 0.25 mm. The distal body 13 includes first support bodies 131 and second support bodies 132, thus forming three unit bodies 130, each of which has an "M"-shaped contour. A "W"-shaped opening 1301 is formed between two adjacent unit bodies 130. The ratio of an area of a single opening 1301 to an area of a region formed by combining the distal body 13 with the openings 1301 is 16%. Reinforcing members 133 are provided. The height of a peak $n_2$ is 1/2 of the height of a peak $n_1$. The sum of outer surface areas of the first support bodies 131 and the sum of outer surface areas of the reinforcing members 133 respectively account for 1.3% and 1.2% of the outer surface area of a cylinder enclosed by the unit bodies 130. An angle $\beta_1$ of each first support body is 75° and an angle $\beta_2$ of each second support body is 90°. The distal body 13 is directly connected to the stent body 11, and the proximal body 12 is connected to the stent body 11 through a second connection rod 15. The length of the second connection rod 15 is 3.5 mm. The length of a waist 112 accounts for 20% of the total length of the valve stent 1. Two ends of the valve stent 1 upwarp. The diameter of the stent body 11 is 20 mm. The ratio of a maximum diameter of the proximal body 12 to the maximum diameter of the stent body and the ratio of the maximum diameter of the distal body 13 to the maximum diameter of the stent body are both 1.2. An angle $\alpha_3$ between a connection line from a vertex of the distal body 13 to a midpoint of the waist 112 and a central axis of the stent body 11 and an angle $\alpha_4$ between a connection line from a vertex of the proximal body 12 to the midpoint of the waist 112 and the central axis of the stent body 11 are both 8°.

The radial support force of the stent body 11 is 30 kPa. The radial support force of the proximal body 12 is 25 kPa. The distal body 13 has a radial support force of 15 kPa and an axial deformation of 3.5 N. The valve stent 1 can be implanted into the pulmonary valve of a dog through an interventional method. The valve stent 1 expands uniformly, can be properly fixed in a lumen, without displacement, and can support the lumen and not affect blood flow in a distal branch blood vessel. At the 12th month, a second valve stent is implanted in situ, and the implantation process is smooth. The expansion of the valve stent is not restricted, and the blood vessel can still be expanded to an expected diameter. Later, the valve stent is withdrawn. It can be observed that the valve stent implanted for the first time has been significantly degraded.

Embodiment 19

This embodiment provides an iron-based absorbable valve stent 1 for a pulmonary valve, which is made of nitrided iron having a tensile strength of 950 MPa and a yield strength of 900 MPa. The valve stent 1 includes a stent body 11, a proximal body 12, and a distal body 13. The stent body 11 is formed by connecting a plurality of porous grid structures. Each porous grid structure in the same circumferential direction is formed by combining 12 support rods. Each stent rod has a wall thickness of 0.15 mm and a rod width of 0.35 mm. The distal body 13 includes first support bodies 131 and second support bodies 132. A rod width of each first support body 131 is partially increased by 1.5 times.

The first support bodies 131 and the second support bodies 132 form two unit bodies 130, each of which has an "M"-shaped contour. A "W"-shaped opening 1301 is formed between two adjacent unit bodies 130. The ratio of the area of a single opening 1301 to the area of a region formed by combining the distal body 13 with the openings 1301 is 25%. Reinforcing members 133 are provided. The height of a peak $n_2$ is 2/3 of the height of a peak $n_1$. The sum of outer surface areas of the first support bodies 131 and the sum of outer surface areas of the reinforcing members 133 respectively account for 1.2% and 0.6% of the outer surface area of a cylinder enclosed by the unit bodies 130. An angle $\beta_1$ of each first support body is 110° and an angle $\beta_2$ of each second support body is 160°. The distal body 13 is directly connected to the stent body 11, and the proximal body 12 is connected to the stent body 11 through a second connection rod 15. The length of the second connection rod is 1.5 mm. The length of a waist 112 accounts for 60% of the total length of the valve stent 1. Two ends of the valve stent 1 upwarp. The diameter of the stent body 11 is 16 mm. The ratio of the maximum diameter of the proximal body 12 to the maximum diameter of the stent body and the ratio of the maximum diameter of the distal body 13 to the maximum diameter of the stent body are respectively 1.8 and 1.4. An angle $\alpha_3$ between a connection line from a vertex of the distal body 13 to a midpoint of the waist 112 and a central axis of the stent body 11 and an angle $\alpha_4$ between a connection line from a vertex of the proximal body 12 to the midpoint of the waist 112 and the central axis of the stent body 11 are respectively 30° and 14°.

The radial support force of the stent body 11 is 6 kPa. The radial support force of the proximal body 12 is 6 kPa. The distal body 13 has a radial support force of 6 kPa and an axial deformation of 1.8 N. The valve stent 1 can be implanted into the pulmonary valve of a dog through an interventional method. The stent expands uniformly, can be properly fixed in a lumen, without displacement, and can support the lumen and not affect blood flow in a distal branch blood vessel. At the 12th month, a second valve stent is implanted in situ, and the implantation process is smooth. The expansion of the valve stent is not restricted, and the blood vessel can still be expanded to an expected diameter. Later, the valve stent is withdrawn. It can be observed that the valve stent implanted for the first time has been significantly degraded.

Embodiment 20

This embodiment provides a valve stent 1 for a pulmonary valve, which is made of cobalt-chromium alloy having a tensile strength of 1500 MPa and a yield strength of 1450 MPa. The valve stent 1 includes a stent body 11, a proximal body 12, and a distal body 13. The stent body 11 is formed by connecting a plurality of porous grid structures. Each porous grid structure in the same circumferential direction is formed by combining 18 support rods. Each stent rod has a wall thickness of 0.4 mm and a rod width of 0.5 mm. The distal body 13 includes first support bodies 131 and second support bodies 132, thus forming three unit bodies 130, each of which has an "M"-shaped contour. A "W"-shaped opening 1301 is formed between two adjacent unit bodies 130. The ratio of the area of a single opening 1301 to the area of a region formed by combining the distal body 13 with the openings 1301 is 10%. Reinforcing members 133 are provided. The height of a peak $n_2$ is 1/2 of the height of a peak $n_1$. The sum of outer surface areas of the first support bodies 131 and the sum of outer surface areas of the reinforcing members 133 respectively account for 2.5% and 2.0% of the outer surface area of a cylinder enclosed by the unit bodies 130. An angle $\beta_1$ of each first support body is 50° and an angle $\beta_2$ of each second support body is 90°. The distal body 13 is directly connected to the stent body 11, and the proximal body 12 is connected to the stent body 11 through a second connection rod 15. The length of the second connection rod 15 is 0.8 mm. The length of a waist 112 accounts for 15% of the total length of the valve stent 1. Two ends of the valve stent 1 upwarp. The diameter of the stent body 11 is 16 mm. The ratio of a maximum diameter of the proximal body 12 to the maximum diameter of the stent body 11 and the ratio of the maximum diameter of the distal body 13 to the maximum diameter of the stent body 11 are both 1.2. An angle $\alpha_3$ between a connection line from a vertex of the distal body 13 to a midpoint of the waist 112 and a central axis of the stent body 11 and an angle $\alpha_4$ between a connection line from a vertex of the proximal body 12 to the midpoint of the waist 112 and the central axis of the stent body 11 are both 7°.

The radial support force of the stent body 11 is 140 kPa. The radial support force of the proximal body 12 is 120 kPa. The distal body 13 has a radial support force of 100 kPa and an axial deformation of 6.2 N. The valve stent 1 can be implanted into the pulmonary valve of a dog through an interventional method. The stent expands uniformly, can be properly fixed in a lumen, without displacement, and can support the lumen and not affect blood flow in a distal branch blood vessel.

Embodiment 21

This embodiment provides an iron-based absorbable valve stent 1 for a pulmonary valve, which is made of iron-manganese alloy having a tensile strength of 1500 MPa and a yield strength of 1450 MPa. The valve stent 1 includes a stent body 11, a proximal body 12, and a distal body 13. The stent body 11 is formed by connecting a plurality of porous grid structures. Each porous grid structure in the same circumferential direction is formed by combining 18 support rods. Each stent rod has a wall thickness of 0.4 mm and a rod width of 0.4 mm. The distal body 13 includes first support bodies 131 and second support bodies 132, thus forming three unit bodies 130, each of which has an "M"-shaped contour. A "W"-shaped opening 1301 is formed between two adjacent unit bodies 130. The ratio of the area of a single opening 1301 to the area of a region formed by combining the distal body 13 with the openings 1301 is 11%. Reinforcing members 133 are provided. The height of a peak $n_2$ is 1/2 of the height of a peak $n_1$. The sum of outer surface areas of the first support bodies 131 and the sum of outer surface areas of the reinforcing members 133 respectively account for 2.0% and 1.5% of the outer surface area of a cylinder enclosed by the unit bodies 130. An angle $\beta_1$ of each first support body is 75° and an angle $\beta_2$ of each second support body is 90°. The distal body 13 is directly connected to the stent body 11, and the proximal body 12 is connected to the stent body 11 through a second connection rod 15. The length of the second connection rod 15 is 0.8 mm. The length of a waist 112 accounts for 15% of the total length of the valve stent. Two ends of the valve stent 1 upwarp. The diameter of the stent body 11 is 16 mm. The ratio of a maximum diameter of the proximal body 12 to the maximum diameter of the stent body and the ratio of the maximum diameter of the distal body 13 to the maximum diameter of the stent body are both 1.2. An angle $\alpha_3$ between a connection line from a vertex of the distal body 13 to a midpoint of the waist 112 and a central axis of the stent body 11 and an angle $\alpha_4$ between a connection line from a vertex of the proximal body 12 to the midpoint of the waist 112 and the central axis of the stent body 11 are both 7°.

The radial support force of the stent body 11 is 120 kPa. The radial support force of the proximal body 12 is 100 kPa. The distal body 13 has a radial support force of 75 kPa and an axial deformation of 5.8 N. The valve stent 1 can be implanted into the pulmonary valve of a dog through an interventional method. The stent expands uniformly, can be properly fixed in a lumen, without displacement, and can support the lumen and not affect blood flow in a distal branch blood vessel. At the 12th month, a second valve stent is implanted in situ, and the implantation process is smooth. The expansion of the valve stent is not restricted by the original valve stent, and the blood vessel can still be expanded to an expected diameter. Later, the valve stent is withdrawn. It can be observed that the valve stent implanted for the first time has been significantly degraded.

Embodiment 22

This embodiment provides a valve stent 1 for a pulmonary valve, which is made of cobalt-chromium alloy having a tensile strength of 1500 MPa and a yield strength of 1450 MPa. The valve stent 1 includes a stent body 11, a proximal body 12, and a distal body 13. The stent body 11 is formed by connecting a plurality of porous grid structures. Each porous grid structure in the same circumferential direction is formed by combining 18 support rods. Each stent rod has a wall thickness of 0.4 mm and a rod width of 0.5 mm. The distal body 13 includes first support bodies 131 and second support bodies 132, thus forming three unit bodies 130, each of which has an "M"-shaped contour. A "W"-shaped opening 1301 is formed between two adjacent unit bodies 130. The ratio of the area of a single opening 1301 to the area of a region formed by combining the distal body 13 with the openings 1301 is 10%. Reinforcing members 133 are provided. The height of a peak $n_2$ is 1/2 of the height of a peak $n_1$. The sum of outer surface areas of the first support bodies 131 and the sum of outer surface areas of the reinforcing members 133 respectively account for 2.5% and 2.0% of the outer surface area of a cylinder enclosed by the unit bodies 130. An angle $\beta_1$ of each first support body is 50° and an angle $\beta_2$ of each second support body is 90°. The distal body 13 is directly connected to the stent body 11, and the proximal body 12 is connected to the stent body 11 through a second connection rod 15. The length of the second connection rod 15 is 0.8 mm. The length of a waist 112 accounts for 15% of the total length of the valve stent 1. Two ends of the valve stent 1 upwarp. The diameter of the stent body 11 is 16 mm. The ratio of the maximum diameter of the proximal body 12 to the maximum diameter of the stent body 11 and the ratio of the maximum diameter of the distal body 13 to the maximum diameter of the stent body 11 are both 1.2. An angle $\alpha_3$ between a connection line from a vertex of the distal body 13 to a midpoint of the waist 112 and a central axis of the stent body 11 and an angle $\alpha_4$ between a connection line from a vertex of the proximal body 12 to the midpoint of the waist 112 and the central axis of the stent body 11 are both 7°.

The radial support force of the stent body 11 is 140 kPa. The radial support force of the proximal body 12 is 120 kPa. The distal body 13 has a radial support force of 100 kPa and an axial deformation of 6.2 N. The valve stent 1 can be implanted into the pulmonary valve of a dog through an interventional method. The stent expands uniformly, can be properly fixed in a lumen, without displacement, and can support the lumen and not affect blood flow in a distal branch blood vessel.

Embodiment 23

This embodiment provides an iron-based absorbable valve stent 1 for a pulmonary valve, which is made of nitrided iron having a tensile strength of 950 MPa and a yield strength of 900 MPa. The valve stent 1 includes a stent body 11, a proximal body 12, and a distal body 13. The stent body 11 is formed by connecting a plurality of porous grid structures 14. Each porous grid structure 14 in the same circumferential direction is formed by combining 24 support rods. Each support rod has a wall thickness of 0.35 mm and a rod width of 0.15 mm. The distal body 13 includes first support bodies 131 and second support bodies 132, thus forming four unit bodies 130, each of which has an "M"-shaped contour. A "W"-shaped opening 1301 is formed between two adjacent unit bodies 130. The ratio of an area of a single opening 1301 to the area of a region formed by combining the distal body 13 with the openings 1301 is 15%. Reinforcing members 133 are provided. The height of a peak $n_2$ is 1/2 of the height of a peak $n_1$. The sum of

outer surface areas of the first support bodies 131 and the sum of outer surface areas of the reinforcing members 133 respectively account for 1.5% and 1.3% of the outer surface area of a cylinder enclosed by the unit bodies 130. An angle $\beta_1$ of each first support body is 40° and an angle $\beta_2$ of each second support body is 110°. The distal body 13 is directly connected to the stent body 11, and the proximal body 12 is connected to the stent body 11 through a second connection rod 15. The length of the second connection rod is 3.5 mm. The length of a waist 112 accounts for 20% of the total length of the valve stent. Two ends of the stent upwarp. The diameter of the stent body 11 is 24 mm. The ratio of the maximum diameter of the proximal body 12 to the maximum diameter of the stent body 11 and the ratio of a maximum diameter of the distal body 13 to the maximum diameter of the stent body 11 are both 1.3. An angle $\alpha_3$ between a connection line from a vertex of the distal body 13 to a midpoint of the waist 112 and a central axis of the stent body 11 and an angle $\alpha_4$ between a connection line from a vertex of the proximal body 12 to the midpoint of the waist 112 and the central axis of the stent body 11 are both 9°.

The radial support force of the stent body 11 is 60 kPa. The radial support force of the proximal body 12 is 45 kPa. The distal body 13 has a radial support force of 25 kPa and an axial deformation of 4.8 N. The valve stent can be implanted into the pulmonary valve of a dog through an interventional method. The stent expands uniformly, can be properly fixed in a lumen, without displacement, and can support the lumen and not affect blood flow in a distal branch blood vessel. At the 12th month, a second valve stent is implanted in situ, and the implantation process is smooth. The expansion of the valve stent is not restricted by the original stent, and the blood vessel can still be expanded to an expected diameter. Later, the valve stent is withdrawn. It can be observed that the valve stent implanted for the first time has been significantly degraded.

Embodiment 24

This embodiment provides an iron-based absorbable valve stent 1 for a pulmonary valve, which is made of nitrided iron having a tensile strength of 950 MPa and a yield strength of 900 MPa. The valve stent 1 includes a stent body 11, a proximal body 12, and a distal body 13. The stent body 11 is formed by connecting a plurality of porous grid structures 14. Each porous grid structure 14 in the same circumferential direction is formed into a closed ring by combining 18 support rods. Each support rod has a wall thickness of 0.29 mm and a rod width of 0.4 mm. The distal body 13 includes first support bodies 131 and second support bodies 132, thus forming three unit bodies 130, each of which has an "M"-shaped contour. A "W"-shaped opening 1301 is formed between two adjacent unit bodies 130. The ratio of the area of a single opening 1301 to the area of a region formed by combining the distal body 13 with the openings 1301 is 17%. Reinforcing members 133 are provided. The height of a peak $n_2$ is 1/2 of the height of a peak $n_1$. The sum of outer surface areas of the first support bodies 131 and the sum of outer surface areas of the reinforcing members 133 respectively account for 1.8% and 1.6% of the outer surface area of a cylinder enclosed by the unit bodies 130. An angle $\beta_1$ of each first support body is 40° and an angle $\beta_2$ of each second support body is 40°. The distal body 13 is directly connected to the stent body 11, and the proximal body 12 is connected to the stent body 11 through a second connection rod 15. The length of the second connection rod 15 is 1.5 mm. The length of a waist 112 accounts for 40% of the total length of the valve stent. Two ends of the valve stent 1 upwarp. The diameter of the stent body 11 is 28 mm. The ratio of the maximum diameter of the proximal body 12 to the maximum diameter of the stent body 11 and the ratio of a maximum diameter of the distal body 13 to the maximum diameter of the stent body are both 1.5. An angle $\alpha_3$ between a connection line from a vertex of the distal body 13 to a midpoint of the waist 112 and a central axis of the stent body 11 and an angle $\alpha_4$ between a connection line from a vertex of the proximal body 12 to the midpoint of the waist 112 and the central axis of the stent body 11 are both 25°.

The radial support force of the stent body 11 is 40 kPa. The radial support force of the proximal body 12 is 30 kPa. The distal body 13 has a radial support force of 15 kPa and an axial deformation of 5.1 N. The valve stent 1 can be implanted into the pulmonary valve of a dog through an interventional method. The stent expands uniformly, can be properly fixed in a lumen, without displacement, and can support the lumen and not affect blood flow in a distal branch blood vessel. At the 12th month, a second valve stent is implanted in situ, and the implantation process is smooth. The expansion of the valve stent is not restricted by the original stent, and the blood vessel can still be expanded to an expected diameter. Later, the valve stent is withdrawn. It can be observed that the valve stent implanted for the first time has been significantly degraded.

Embodiment 25

This embodiment provides an iron-based absorbable valve stent 1 for a pulmonary valve, which is made of nitrided iron having a tensile strength of 900 MPa and a yield strength of 850 MPa. The valve stent 1 includes a stent body 11, a proximal body 12, and a distal body 13. The stent body 11 is formed by connecting a plurality of porous grid structures 14. Each porous grid structure 14 in the same circumferential direction is formed into a closed ring by combining 18 support rods. Each support rod has a wall thickness of 0.4 mm and a rod width of 0.5 mm. The distal body 13 includes first support bodies 131 and second support bodies 132, thus forming three unit bodies 130, each of which has an "M"-shaped contour. A "W"-shaped opening 1301 is formed between two adjacent unit bodies 130. The ratio of the area of a single opening 1301 to the area of a region formed by combining the distal body 13 with the openings 1301 is 18%. Reinforcing members 133 are provided. The height of a peak $n_2$ is 1/2 of the height of a peak $n_1$. The sum of outer surface areas of the first support bodies 131 and the sum of outer surface areas of the reinforcing members 133 respectively account for 1.6% and 1.2% of the outer surface area of a cylinder enclosed by the unit bodies 130. An angle $\beta_1$ of each first support body is 60° and an angle $\beta_2$ of each second support body is 60°. The distal body 13 is directly connected to the stent body 11, and the proximal body 12 is connected to the stent body 11 through a second connection rod 15. The length of the second connection rod 15 is 2.5 mm. The length of a waist 112 accounts for 25% of the total length of the valve stent. Two ends of the valve stent 1 upwarp. The diameter of the stent body 11 is 30 mm. The ratio of the maximum diameter of the proximal body 12 to the maximum diameter of the stent body 11 and the ratio of a maximum diameter of the distal body 13 to the maximum diameter of the stent body are both 1.2. An angle $\alpha_3$ between a connection line from a vertex of the distal body 13 to a midpoint of the waist 112 and a central axis of the stent body 11 and an angle $\alpha_4$ between a connection line from a vertex of the proximal body 12 to the midpoint of the waist 112 and the central axis of the stent body 11 are both 11°.

The radial support force of the stent body 11 is 45 kPa. The radial support force of the proximal body is 35 kPa. The distal body has a radial support force of 20 kPa and an axial deformation of 6.1 N. The stent can be implanted into the pulmonary valve of a dog through an interventional method. The stent expands uniformly, can be properly fixed in a lumen, without displacement, and can support the lumen and not affect blood flow in a distal branch blood vessel. At the 12th month, a second valve stent is implanted in situ, and the implantation process is smooth. The expansion of the valve stent is not restricted by the original stent, and the blood vessel can still be expanded to an expected diameter. Later, the valve stent is withdrawn. It can be observed that the valve stent implanted for the first time has been significantly degraded.

Embodiment 26

This embodiment provides an iron-based absorbable valve stent 1 for a pulmonary valve, which is made of nitrided iron having a tensile strength of 850 MPa and a yield strength of 800 MPa. The valve stent 1 includes a stent body 11, a proximal body 12, and a distal body 13. The stent body 11 is formed by connecting a plurality of porous grid structures 14. Each porous grid structure 14 in the same circumferential direction is formed into a closed ring by combining 18 support rods. Each support rod has a thickness of 0.18 mm and a rod width of 0.3 mm. The distal body 13 includes first support bodies 131 and second support bodies 132, thus forming three unit bodies 130, each of which has an "M"-shaped contour. A "W"-shaped opening 1301 is formed between two adjacent unit bodies 130. The ratio of the area of a single opening 1301 to the area of a region formed by combining the distal body 13 with the openings 1301 is 15%. Reinforcing members 133 are provided. The height of a peak $n_2$ is 1/2 of the height of a peak $n_1$. The sum of outer surface areas of the first support bodies 131 and the sum of outer surface areas of the reinforcing members 133 respectively account for 1.4% and 1.3% of the outer surface area of a cylinder enclosed by the unit bodies 130. An angle $\beta_1$ of each first support body is 60° and an angle $\beta_2$ of each second support body is 170°. The distal body 13 is directly connected to the stent body 11, and the proximal body 12 is connected to the stent body 11 through a second connection rod 15. The length of the second connection rod 15 is 2.8 mm. The length of a waist 112 accounts for 15% of the total length of the valve stent. Two ends of the valve stent 1 upwarp. The diameter of the stent body 11 is 16 mm. The ratio of the maximum diameter of the proximal body 12 to the maximum diameter of the stent body 11 and the ratio of a maximum diameter of the distal body 13 to the maximum diameter of the stent body are both 1.1. An angle $\alpha_3$ between a connection line from a vertex of the distal body 13 to a midpoint of the waist 112 and a central axis of the stent body 11 and an angle $\alpha_4$ between a connection line from a vertex of the proximal body 12 to the midpoint of the waist 112 and the central axis of the stent body 11 are both 3°.

The radial support force of the stent body 11 is 12 kPa. The radial support force of the proximal body 12 is 8 kPa. The distal body 13 has a radial support force of 6 kPa and an axial deformation of 1.6 N. The valve stent 1 can be implanted into the pulmonary valve of a dog through an interventional method. The stent expands uniformly, can be properly fixed in a lumen, without displacement, and can support the lumen and not affect blood flow in a distal branch blood vessel. At the 12th month, a second valve stent is implanted in situ, and the implantation process is smooth. The expansion of the valve stent is not restricted by the original stent, and the blood vessel can still be expanded to an expected diameter. Later, the valve stent is withdrawn. It can be observed that the valve stent implanted for the first time has been significantly degraded.

Embodiment 27

This embodiment provides an iron-based absorbable valve stent 1 for a pulmonary valve, which is made of nitrided iron having a tensile strength of 850 MPa and a yield strength of 800 MPa. The valve stent 1 includes a stent body 11, a proximal body 12, and a distal body 13. The stent body 11 is formed by connecting a plurality of porous grid structures. Each porous grid structure in the same circumferential direction is formed into a closed ring by combining 18 support rods. Each support rod has a wall thickness of 0.28 mm and a rod width of 0.18 mm. The distal body 13 includes first support bodies 131 and second support bodies 132, thus forming three unit bodies 130, each of which has an "M"-shaped contour. A "W"-shaped opening 1301 is formed between two adjacent unit bodies 130. The ratio of the area of a single opening 1301 to the area of a region formed by combining the distal body 13 with the openings 1301 is 13%. Reinforcing members 133 are provided. The height of a peak $n_2$ is 1/3 of the height of a peak $n_1$. The sum of outer surface areas of the first support bodies 131 and the sum of outer surface areas of the reinforcing members 133 respectively account for 1.0% and 0.9% of the outer surface area of a cylinder enclosed by the unit bodies 130. An angle $\beta_1$ of each first support body is 60° and an angle $\beta_2$ of each second support body is 179°. The distal body 13 is directly connected to the stent body 11, and the proximal body 12 is connected to the stent body 11 through a second connection rod 15. The length of the second connection rod 15 is 2.8 mm. The length of a waist 112 accounts for 15% of a total length of the valve stent. Two ends of the valve stent 1 upwarp. The diameter of the stent body 11 is 16 mm. The ratio of the maximum diameter of the proximal body 12 to the maximum diameter of the stent body 11 and the ratio of a maximum diameter of the distal body 13 to the maximum diameter of the stent body are both 1.3. An angle $\alpha_3$ between a connection line from a vertex of the distal body 13 to a midpoint of the waist 112 and a central axis of the stent body 11 and an angle $\alpha_4$ between a connection line from a vertex of the proximal body 12 to the midpoint of the waist 112 and the central axis of the stent body 11 are both 10°.

The radial support force of the stent body 11 is 50 kPa. The radial support force of the proximal body 12 is 40 kPa. The distal body 13 has a radial support force of 30 kPa and an axial deformation of 2.6 N. The valve stent 1 can be implanted into the pulmonary valve of a dog through an interventional method. The stent expands uniformly, can be properly fixed in a lumen, without displacement, and can support the lumen and not affect blood flow in a distal branch blood vessel. At the 12th month, a second valve stent is implanted in situ, and the implantation process is smooth. The expansion of the valve stent is not restricted by the original stent, and the blood vessel can still be expanded to an expected diameter. Later, the valve stent is withdrawn. It can be observed that the valve stent implanted for the first time has been significantly degraded.

Embodiment 28

This embodiment provides a magnesium-based absorbable valve stent 1 for a pulmonary valve, which is made of magnesium alloy having a tensile strength of 400 MPa and a yield strength of 350 MPa. The valve stent 1 includes a stent body 11, a proximal body 12, and a distal body 13. The stent body 11 is formed by connecting a plurality of porous grid structures 14. Each porous grid structure 14 in the same circumferential direction is formed into a closed ring by combining 18 support rods. Each support rod has a wall thickness of 0.3 mm and a rod width of 0.3 mm. The distal body 13 includes first support bodies 131 and second support bodies 132. Particularly, the wall thickness and rod width of each first support body 131 is partially increased by 1.3 times. The first support bodies 131 and the second support bodies 132 form three unit bodies 130, each of which has an "M"-shaped contour. A "W"-shaped opening 1301 is formed between two adjacent unit bodies 130. The ratio of the area of a single opening 1301 to the area of a region formed by combining the distal body 13 with the openings 1301 is 14%. Reinforcing members 133 are provided. The height of a peak $n_2$ is 1/3 of the height of a peak $n_1$. The sum of outer surface areas of the first support bodies 131 and the sum of outer surface areas of the reinforcing members 133 respectively account for 1.6% and 1.3% of the outer surface area of a cylinder enclosed by the unit bodies 130. An angle $\beta_1$ of each first support body is 40° and an angle $\beta_2$ of each second support body is 50°. The distal body 13 is directly connected to the stent body 11, and the proximal body 12 is connected to the stent body 11 through a second connection rod 15. The length of the second connection rod 15 is 1.5 mm. The length of a waist 11 accounts for 15% of a total length of the valve stent. Two ends of the valve stent 1 upwarp. The diameter of the stent body 11 is 16 mm. The ratio of the maximum diameter of the proximal body 12 to the maximum diameter of the stent body 11 and the ratio of a maximum diameter of the distal body 13 to the maximum diameter of the stent body are both 1.3. An angle $\alpha_3$ between a connection line from a vertex of the distal body 13 to a midpoint of the waist 112 and a central axis of the stent body 11 and an angle $\alpha_4$ between a connection line from a vertex of the proximal body 12 to the midpoint of the waist 112 and the central axis of the stent body 11 are both 10°.

The radial support force of the stent body 11 is 12 kPa. The radial support force of the proximal body 12 is 8 kPa. The distal body 13 has a radial support force of 8 kPa and an axial deformation of 2.1 N. The valve stent 1 can be implanted into the pulmonary valve of a dog through an interventional method. The stent expands uniformly, can be properly fixed in a lumen, without displacement, and can support the lumen and not affect blood flow in a distal branch blood vessel. At the 12th month, a second valve stent is implanted in situ, and the implantation process is smooth. The expansion of the valve stent is not restricted by the original stent, and the blood vessel can still be expanded to an expected diameter. Later, the valve stent is withdrawn. It can be observed that the valve stent implanted for the first time has been significantly degraded.

Embodiment 29

This embodiment provides a magnesium-based absorbable valve stent 1 for a pulmonary valve, which is made of magnesium alloy having a tensile strength of 500 MPa and a yield strength of 400 MPa.

The valve stent 1 includes a stent body 11, a proximal body 12, and a distal body 13. The stent body 11 is formed by connecting a plurality of porous grid structures 14. Each porous grid structure 14 in the same circumferential direction is formed into a closed ring by combining 18 support rods. Each stent rod has a wall thickness of 0.3 mm and a rod width of 0.28 mm. The distal body 13 includes first support bodies 131 and second support bodies 132. Particularly, the wall thickness and rod width of each first support body 131 is partially increased by 1.5 times. The first support bodies 131 and the second support bodies 132 form three unit bodies 130, each of which has an "M"-shaped contour. A "W"-shaped opening 1301 is formed between two adjacent unit bodies 130. The ratio of the area of a single opening 1301 to the area of a region formed by combining the distal body 13 with the openings 1301 is 13%. Reinforcing members 133 are provided. The height of a peak $n_2$ is 1/3 of the height of a peak $n_1$. The sum of outer surface areas of the first support bodies 131 and the sum of outer surface areas of the reinforcing members 133 respectively account for 1.9% and 1.3% of the outer surface area of a cylinder enclosed by the unit bodies 130. An angle $\beta_1$ of each first support body is 75° and an angle $\beta_2$ of each second support body is 90°. The distal body 13 is directly connected to the stent body 11, and the proximal body 12 is connected to the stent body 11 through a second connection rod 15. The length of the second connection rod 15 is 2.8 mm. The length of a waist 112 accounts for 15% of the total length of the valve stent. Two ends of the valve stent 1 upwarp. The diameter of the stent body 11 is 16 mm. The ratio of the maximum diameter of the proximal body 12 to the maximum diameter of the stent body 11 and the ratio of the maximum diameter of the distal body 13 to the maximum diameter of the stent body are both 1.3. An angle $\alpha_3$ between a connection line from a vertex of the distal body 13 to a midpoint of the waist 112 and a central axis of the stent body 11 and an angle $\alpha_4$ between a connection line from a vertex of the proximal body 12 to the midpoint of the waist 112 and the central axis of the stent body 11 are both 10°.

The radial support force of the stent body 11 is 12 kPa. The radial support force of the proximal body 12 is 8 kPa. The distal body 13 has a radial support force of 10 kPa and an axial deformation of 2.6 N. The valve stent 1 can be implanted into the pulmonary valve of a dog through an interventional method. The stent expands uniformly, can be properly fixed in a lumen, without displacement, and can support the lumen and not affect blood flow in a distal branch blood vessel. At the 12th month, a second valve stent is implanted in situ, and the implantation process is smooth. The expansion of the valve stent is not restricted by the original stent, and the blood vessel can still be expanded to an expected diameter. Later, the valve stent is withdrawn. It can be observed that the valve stent implanted for the first time has been significantly degraded.

To further describe technical effects of a degradable valve stent in the embodiments of the application, comparative examples are now exemplified for supplementary instruction.

Comparative example 1

The design of a valve stent for a pulmonary valve in this comparative example is basically the same as that in Embodiment 1. A difference is that the stent in this comparative example is made of a non-degradable cobalt-chromium material.

The stent has a radial support force of 45 kPa, an axial deformation resistance of 3.8 N, and a resistance to parallel plate compression of 0.26 N/mm. The stent can be implanted into the pulmonary valve of a dog through an interventional method. The stent can be properly fixed in a lumen, without displacement, and can support the lumen and not affect blood flow in a distal branch blood vessel. At the 12th month, a second stent is implanted in situ. During expansion of the stent, since the first stent is not deconstructed, restriction is caused. It is difficult to expand the second implanted stent, and expansion to a rated diameter fails. The blood vessel is not expanded to an expected diameter, and reimplantation cannot meet the surgical expectation.

Comparative example 2

The design of a valve stent for a pulmonary valve in this comparative example is basically the same as that in Embodiment 6. A difference is that the wall thickness of each support rod on the stent body 11 is 0.08 mm; the coverage rate of the stent body 11 is 2.1%; and the coverage rate of the distal body 13 is 0.8%.

In this design, the stent has a radial support force of 4 kPa, an axial deformation resistance of 0.2 N, and a resistance to parallel plate compression of 0.03 N/mm. When the stent is implanted into the aortic valve of a dog through an interventional method, the stent is collapsed by the blood vessel due to its excessively low radial support force, axial deformation resistance, and resistance to parallel plate compression, and cannot effectively support the blood vessel. After the stent is withdrawn, it is found that the stent is flattened and undergoes axial deformation.

Comparative example 3

The design of a valve stent for a pulmonary valve in this comparative example is basically the same as that in Embodiment 1. A difference is that the wall thickness of each support rod on the stent body 11 is 0.50mm; the coverage rate of the stent body 11 is 20%; and the coverage rate of the distal body 13 is 8%.

In this design, the stent has a radial support force of 160 kPa, an axial deformation resistance of 8.2 N, and a resistance to parallel plate compression of 1.1 N/mm. The stent can be implanted into the pulmonary valve of a dog through an interventional method. The stent can be implanted into the pulmonary valve of a dog through an interventional method. The stent can be properly fixed in a lumen, without displacement, and can support the lumen and not affect blood flow in a distal branch blood vessel. However, the stent has a wall thickness that is too high and a coverage rate that is too high. At the 12th month, a second valve stent is implanted in situ. The expansion of the valve stent is not restricted, and the blood vessel fails to be completely expanded to an expected diameter. Later, the valve stent is withdrawn. It can be observed that the valve stent implanted for the first time has been significantly degraded. However, because the stent has a wall thickness and a coverage rate that are too high, the stent does not satisfy the degree of expected deconstruction, indicating that the secondary stent implantation fails.

Comparative example 4

The design of a valve stent for a pulmonary valve in this embodiment is basically the same as that in Embodiment 14. A difference is that the distal body in this embodiment is not provided with a unit body with an "M"-shaped contour, and no "W"-shaped opening is formed.
The stent can be implanted into the pulmonary valve of a dog through an interventional method. The stent can be properly fixed in a lumen, without displacement, and can support the lumen, but the distal end of the stent affects blood flow in a distal branch blood vessel, so that the blood flow in the branch blood vessel is reduced.

Comparative example 5

The design of a valve stent for a pulmonary valve in this embodiment is basically the same as that in Embodiment 14. A difference is that the two ends of the stent in this implementation do not upwarp. The distal body, the proximal body, and the stent body of the stent have the same diameters.
The stent can be implanted into the pulmonary valve of a dog through an interventional method. However, it is hard to fix the stent in a lumen, and the stent easily moves.

Comparative example 6

The design of a valve stent for a pulmonary valve in this embodiment is basically the same as that in Embodiment 15. A difference is that no reinforcing member is provided in this implementation.
The radial support force of the stent body of the stent is 6 kPa. The radial support force of the proximal body is 6 kPa. The distal body has a radial support force of 6 kPa and an axial deformation of only 0.5 N. The stent can be implanted into the pulmonary valve of a dog through an interventional method. The stent can be properly fixed in a lumen, without displacement, and can support the lumen and not affect blood flow in a distal branch blood vessel. However, the distal body is twisted to puncture the blood vessel because of axial deformation.

Comparative example 7

The design of a valve stent for a pulmonary valve in this embodiment is basically the same as that in Embodiment 15. A difference is that in this implementation the angle $\beta_1$ of each first support body is 30°, and the angle $\beta_2$ of each second support body is 30°.
The radial support force of the stent body of the stent is only 4 kPa. The radial support force of the proximal body is only 3 kPa. The distal body has a radial support force of only 2 kPa and an axial deformation of 1.6 N. The stent can be implanted into the pulmonary valve of a dog through an interventional method. The stent can be properly fixed in a lumen, without displacement. However, the stent has a low support force and cannot support the blood vessel. After balloon deflation, the stent is collapsed by the blood vessel.

Comparative example 8

The design of a valve stent for a pulmonary valve in this embodiment is basically the same as that in Embodiment 14. A difference is that the stent in this implementation is not in a fully closed design.
The radial support force of the stent body of the stent is 28 kPa. The radial support force of the proximal body is 20 kPa. The distal body has a radial support force of 12 kPa and an axial deformation of 2.6 N. The stent can be implanted into the pulmonary valve of a dog through an interventional method. The stent can be properly fixed in a lumen, without displacement. However, the stent is not expanded uniformly, leading to non-uniform distribution of the valve, so that the valve cannot be completely closed, and regurgitation occurs.
The foregoing descriptions are merely preferred specific implementations of the present application, but are not intended to limit the protection scope of the present application. Any variation or replacement readily figured out by a person skilled in the art within the technical scope disclosed in the present application shall fall within the protection scope of the present application.

**Claims**

1. A degradable valve stent, comprising a stent body which is constructed in a preset shape, and a proximal body and a distal body that are respectively connected to two axial ends of the stent body, wherein the stent body, the proximal body, and the distal body overall form a circumferentially closed ringlike structure; and each of the stent body, the proximal body, and the distal body is at least partially degradable, so that the circumferentially closed ringlike structure of the degradable valve stent is deconstructed.

2. The degradable valve stent according to claim 1, wherein the proportion of the total outer surface area of the degradable portion of the degradable valve stent to the total outer surface area of the entire degradable valve stent is greater than or equal to 0.5%; or the proportion of the total outer surface area of the degradable portion of the degradable valve stent to the total outer surface area of the entire degradable valve stent is greater than or equal to 5%; or the proportion of the total outer surface area of the degradable portion of the degradable valve stent to the total outer surface area of the entire degradable valve stent is greater than or equal to 99%.

3. The degradable valve stent according to claim 1, wherein the radial support force of the degradable valve stent is [6 kPa, 140 kPa]; or the radial support force of the degradable valve stent is [10 kPa, 120 kPa]; or the radial support force of the degradable valve stent is [12 kPa, 100 kPa].

4. The degradable valve stent according to claim 1, wherein the coverage rate of the degradable valve stent is [4%, 18%]; the coverage rate of the degradable valve stent is [4%, 15%]; the coverage rate of the degradable valve stent is [4%, 8%).

5. The degradable valve stent according to claim 1, wherein the wall thickness of the degradable valve stent is 0.10 mm to 0.40 mm; the wall thickness of the degradable valve stent is 0.15 mm to 0.4 mm; the wall thickness of the degradable valve stent is 0.18 mm to 0.35 mm.

6. The degradable valve stent according to claim 1, wherein the resistance to parallel plate compression of the degradable valve stent is not less than 0.05 N/mm.

7. The degradable valve stent according to claim 1, wherein the degradable valve stent is a valve stent for a pulmonary valve, with a radial support force of [10 kPa, 80 kPa], a coverage rate of [4%, 13%], and a wall thickness of 0.15 mm to 0.35 mm; or the degradable valve stent is a valve stent for an aortic valve, with a radial support force of [30 kPa, 120 kPa], a coverage rate of [5%, 15%], and a wall thickness of 0.18 mm to 0.40 mm; or the degradable valve stent is a valve stent for a mitral valve, with a radial support force of [30 kPa, 100 kPa], a coverage rate of [5%, 13%], and a wall thickness of 0.18 mm to 0.35 mm; or the degradable valve stent is a valve stent for a tricuspid valve, with a radial support force of [30 kPa, 110 kPa], a coverage rate of [5%, 14%], and a wall thickness of 0.18 mm to 0.38 mm.

8. The degradable valve stent according to claim 1, wherein the degradable valve stent is a valve stent for a pulmonary valve, with a radial support force of [6 kPa, 80 kPa], a coverage rate of [4%, 13%], and a wall thickness of 0.15 mm to 0.35 mm; or the degradable valve stent is a valve stent for an aortic valve, with a radial support force of [30 kPa, 140 kPa], a coverage rate of [5%, 18%], and a wall thickness of 0.18 mm to 0.40 mm; or the degradable valve stent is a valve stent for a mitral valve, with a radial support force of [12 kPa, 100 kPa], a coverage rate of [5%, 13%], and a wall thickness of 0.15 mm to 0.35 mm; or the degradable valve stent is a valve stent for a tricuspid valve, with a radial support force of [30 kPa, 110 kPa], a coverage rate of [5%, 14%], and a wall thickness of 0.15 mm to 0.38 mm.

9. The degradable valve stent according to claim 1, wherein each of the stent body, the proximal body, and/or the distal body comprises a plurality of porous grid structures, and the porous grid structures are fully closed.

10. The degradable valve stent according to claim 9, wherein each porous grid structure comprises a peak and a trough; an angle $\alpha_1$ between two adjacent support rods that form the peak or the trough on the stent body is 50° to 160°; an angle $\alpha_2$ between two adjacent support rods that form the peak or the trough of the proximal body and/or the distal body is 40° to 179°.

11. The degradable valve stent according to claim 10, wherein in a circumferential direction of the stent body, a total number of 12 to 48 support rods are provided on porous grid structures surrounding the same position.

12. The degradable valve stent according to claim 10, wherein in a direction from the proximal body to the distal body, the peak of an $n^{th}$ porous grid structure is connected to the trough of an $(n+1)^{th}$ porous grid structure directly or through a first connection rod, wherein n is greater than or equal to 1; the length of the first connection rod is 0.1 mm to 3.0 mm.

13. The degradable valve stent according to claim 1, wherein the coverage rate of the distal body is less than or equal to 6%.

14. The degradable valve stent according to any one of claims 1 to 13, wherein an axial deformation resistance of the degradable valve stent is not less than 0.3 N; and the axial deformation resistance of the degradable valve stent is not less than 1 N.

**15.** The degradable valve stent according to any one of claims 1 to 13, wherein a length of the stent body is 15% to 75% of a total length of the valve stent; and the length of the stent body is 20% to 65% of the total length of the valve stent.

**16.** A valve stent for a pulmonary valve, comprising a tubular stent body, and a proximal body and a distal body that are respectively connected to two axial ends of the stent body, wherein the stent body comprises a plurality of porous grid structures; the distal body comprises a plurality of unit bodies, each of which has an "M"-shaped contour; wherein m unit bodies are comprised; and m is a natural number greater than or equal to 2.

**17.** A valve stent for a pulmonary valve, comprising the degradable valve stent according to any one of claims 1 to 6 and 9 to 15, wherein the distal body comprises a plurality of unit bodies, each of which has an "M"-shaped contour; wherein m unit bodies are comprised; and m is a natural number greater than or equal to 2.

**18.** The valve stent according to claim 16 or 17, wherein each unit body comprises a first support body and a second support body, and one end of the first support body and one end of the second support body are connected to each other, and are connected to the stent body.

**19.** The valve stent according to claim 16 or 17, wherein a "W"-shaped opening is formed between two adjacent unit bodies, and the ratio of the area of a single opening to the area of a region formed by combining the distal body and the openings is greater than or equal to 10%.

**20.** The valve stent according to claim 18, wherein the first support body is bent to form a peak $n_1$; the second support body is bent in the same direction to form a peak $n_2$; the height of the peak $n_2$ of the second support body is less than or equal to 2/3 of the height of the peak $n_1$ of adjacent first support body on each side.

**21.** The valve stent according to claim 18, wherein the width of the first support body is 1.0 to 3.5 times the width of the second support body.

**22.** The valve stent according to claim 16 or 17, wherein each unit body further comprises a reinforcing member; and the reinforcing member is arranged between the stent body and the distal body, the reinforcing member is at least connected to the first support body.

**23.** The valve stent according to claim 18, wherein the percentage of the sum of outer surface areas of the first support bodies to the side surface area of a cylinder enclosed by the unit bodies is less than or equal to 2.5%; the percentage of the sum of outer surface areas of the reinforcing members to the side surface area of the cylinder enclosed by the unit bodies is less than or equal to 2.0%.

**24.** The valve stent according to claim 18, wherein the angle formed by bending of each first support body is [40°, 110°] and/or the angle formed by bending of each second support body is [40°, 179°].

**25.** The valve stent according to claim 16 or 17, wherein the stent body has a waist with a consistent diameter, and a connection portion that extends from an edge of the waist toward the proximal body and/or the distal body and has a gradually increasing diameter; the proximal body and the distal body are respectively connected to corresponding connection portions.

**26.** The valve stent according to claim 25, wherein the length of the waist is 15% to 60% of the total length of the valve stent; the length of the waist is 20% to 40% of the total length of the valve stent.

**27.** The valve stent according to any one of claims 16 to 26, wherein the radial support force of the stent body is [6 kPa, 140 kPa]; the radial support force of the proximal body is [6 kPa, 120 kPa]; the radial support force of the distal body is [6 kPa, 100 kPa]; the deformation resistance of the distal body is greater than or equal to 1.5 N.

**28.** The degradable valve stent according to any one of claims 1 to 15 or the valve stent for the pulmonary valve according to any one of claims 16 to 27, wherein the angle between a connection line from a vertex of the distal body to a midpoint of the stent body and a central axis of the stent body is $a_3$; an angle between a connection line from a vertex of the proximal body to the midpoint of the stent body and the central axis of the stent body is $\alpha_4$; and $\alpha_3$ or $\alpha_4$ is within (0°, 30°], wherein $\alpha_3$ is equal to $\alpha_4$, or $\alpha_3$ is not equal to $\alpha_4$, or $\alpha_3$ is less than $\alpha_4$.

**29.** The degradable valve stent according to any one of claims 1 to 15 or the valve stent for the pulmonary valve according to any one of claims 16 to 27, wherein the ratio of the maximum diameter of the proximal body and/or the distal body to a minimum diameter of the stent body is [1, 1.8]: 1; and the ratio of the maximum diameter of the proximal body and/or the distal body to the minimum diameter of the stent body is (1, 1.8]: 1.

**30.** The degradable valve stent according to any one of claims 1 to 15 or the valve stent for the pulmonary valve according to any one of claims 16 to 27, wherein the proximal body and/or the distal body are/is connected to the stent body directly or through a second connection rod.

31. The valve stent according to claim 30, wherein the length of the second connection rod is 0.8 mm to 4.0 mm.

32. The degradable valve stent according to claim 1 or the valve stent for the pulmonary valve according to claim 16 or 17, wherein the yield strength of a base material of the valve stent is 350 MPa to 1450 MPa; and/or a tensile strength of the base material of the valve stent is 400 MPa to 1500 MPa.

33. The degradable valve stent according to claim 1 or the valve stent for the pulmonary valve according to claim 16 or 17, wherein the material of a degradable portion of the valve stent is at least one of metal or non-metal; wherein, the metal at least comprises any one of pure iron, iron alloy, pure magnesium, magnesium alloy, pure zinc, or zinc alloy; the non-metal at least comprises any one of degradable polyester, degradable polyanhydride, and a bi- or multi-element degradable copolymer formed by copolymerizing monomers corresponding to the degradable polyester and the degradable polyanhydride; the degradable polyester comprises at least one of polylactic acid, polyglycolic acid, poly(lactide-co-glycolide), polycaprolactone, polylactide, polyglycolide, polycaprolactide, polyhydroxyalkanoate, polyacrylate, polybutylene succinate, poly($\beta$-hydroxybutyrate), poly(ethylene adipate), and a polyhydroxybutyrate-valerate copolymer; the degradable polyanhydride comprises at least one of poly(1,3-bis(p-carboxyphenoxy)propane-sebacic acid), poly(erucic acid dimer-sebacic acid), or poly(fumaric acid-sebacic acid); or the non-metal at least comprises degradable polyamino acid or a degradable tyrosine-derived polymer; the degradable polyamino acid comprises at least one of polyglycine, polyalanine, polyvaline, polyleucine, polyisoleucine, polymethionine, polyproline, polytryptophan, polyserine, polytyrosine, polycysteine, polyphenylalanine, polyasparagine, polyglutamine, polythreonine, polyarginine, polyhistidine, polyselenocysteine, polypyrroline, poly-glutamic acid, poly-aspartic acid, polyornithine, polylysine and derivatives thereof; and the degradable tyrosine-derived polymer comprises methyl (2R)-2-amino-3-(2-chloro-4-hydroxyphenyl)propanoate, D-tyrosine ethyl ester hydrochloride, methyl (2R)-2-amino-3-(2,6-dichloro-3,4-dimethoxyphenyl)propanoate H-D-Tyr(TBU)-allyl ester HCl, methyl (2R)-2-amino-3-(3-chloro-4,5-dimethoxyphenyl)propanoate, methyl (2R)-2-amino-3-(2-chloro-3-hydroxy-4-methoxyphenyl)propanoate, methyl (2R)-2-amino-3-(4-[(2-chloro-6-fluorophenyl)methoxy]phenyl)propanoate, methyl (2R)-2-amino-3-(2-chloro-3,4-dimethoxyphenyl)propanoate, methyl (2R)-2-amino-3-(3-chloro-5-fluoro-4-hydroxyphenyl)propanoate, 2-(acetylamin)-2(4-[(2-chloro-6-fluorobenzyl)oxy]benzyl)diethyl malonate, methyl (2R)-2-amino-3-(3-chloro-4-methoxyphenyl)propanoate, methyl (2R)-2-amino-3-(3-chloro-4-hydroxy-5-methoxyphenyl)propanoate, methyl (2R)-2-amino-3-(2,6-dichloro-3-hydroxy-4-methoxyphenyl)propanoate, methyl (2R)-2-amino-3-(3-chloro-4-hydroxyphenyl)propanoate, H-DL-tyr-OME HCl, H-3,5-diiodo-tyr-OME HCl, H-D-3,5-diiodo-tyr-OME HCl, H-D-tyr-OMEHCl, D-tyrosine methyl ester hydrochloride, D-tyrosine-ome HCl, D-tyrosine methyl ester hydrochloride, H-D-tyr-OMe-HCl, D-tyrosine methyl ester HCl, H-D-Tyr-OMe-HCl, (2R)-2-amino-3-(4-hydroxyphenyl) propanoic acid, methyl (2R)-2-amino-3-(4-hydroxyphenyl)hydrochloride, methyl (2R)-2-amino-3-(4-hydroxyphenyl)propanoate hydrochloride, methyl (2R)-2-aminoalkyl-3-(4-hydroxyphenyl)propanoate hydrochloride, 3-chloro-L-tyrosine, 3-nitro-L-tyrosine, 3-nitro-L-tyrosine ethyl ester hydrochloride, DL-m-tyrosine, DL-o-tyrosine, Boc-Tyr(3, 5-I2)-OSu, Fmoc-tyr(3-NO$_2$)-OH, $\alpha$-methyl-L-tyrosine, $\alpha$-methyl-D-tyrosine, and $\alpha$-methyl-DL-tyrosine.

34. A pulmonary valve, comprising a valve leaflet and the valve stent for a pulmonary valve according to any one of claims 16 to 33, wherein the valve leaflet is connected to the valve stent for a pulmonary valve.

35. A heart valve, comprising a valve leaflet and the degradable valve stent according to any one of claims 1 to 15 and 28 to 33, wherein the valve leaflet is connected to the degradable valve stent.

**FIG. 1**

**FIG. 2**

**FIG. 3**

FIG. 4

**FIG. 5**

**FIG. 6**

**FIG. 7**

**FIG. 8**

**FIG. 9**

## INTERNATIONAL SEARCH REPORT

| International application No. |
|---|
| **PCT/CN2024/108211** |

| A. | CLASSIFICATION OF SUBJECT MATTER |
|---|---|

A61F2/24(2006.01)i

According to International Patent Classification (IPC) or to both national classification and IPC

| B. | FIELDS SEARCHED |
|---|---|

Minimum documentation searched (classification system followed by classification symbols)

IPC: A61F

Documentation searched other than minimum documentation to the extent that such documents are included in the fields searched

Electronic data base consulted during the international search (name of data base and, where practicable, search terms used)

CNTXT, WPABSC, ENTXTC, DWPI: 瓣膜, 瓣, 心脏, 肺动脉, 框架, 框体, 框, 支架, 支撑架, 降解, 吸收, 分解, 水解, 溶解, 解聚, valve, heart, frame, support, brace, bracket, degrade, bioabsorbable

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 113164258 A (EDWARDS LIFESCIENCES CORP.) 23 July 2021 (2021-07-23) description, paragraphs [0123]-[0178], and figures 1A-16E | 1-35 |
| X | CN 114681133 A (SHANGHAI NEWMED MEDICAL CO., LTD.) 01 July 2022 (2022-07-01) description, paragraphs [0057]-[0093], and figures 1-12 | 1-35 |
| X | US 2021236688 A1 (UNIVERSITY OF PITTSBURGH OF THE COMMONWEALTH SYSTEM OF HIGHER EDUCATION et al.) 05 August 2021 (2021-08-05) description, paragraphs [0109]-[0187], and figures 1-8C | 1-35 |
| A | CN 102764169 A (VENUS MEDTECH (HANGZHOU) INC.) 07 November 2012 (2012-11-07) entire document | 1-35 |
| A | CN 112107393 A (FUWAI HOSPITAL CHINESE ACADEMY OF MEDICAL SCIENCES) 22 December 2020 (2020-12-22) entire document | 1-35 |
| A | US 2016279297 A1 (CONCIEVALVE LLC) 29 September 2016 (2016-09-29) entire document | 1-35 |

☐ Further documents are listed in the continuation of Box C.  ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **06 November 2024** | **12 November 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

| INTERNATIONAL SEARCH REPORT | | | | | | | International application No. |
|---|---|---|---|---|---|---|---|
| **Information on patent family members** | | | | | | | **PCT/CN2024/108211** |

| Patent document cited in search report | | | Publication date (day/month/year) | Patent family member(s) | | | Publication date (day/month/year) |
|---|---|---|---|---|---|---|---|
| CN | 113164258 | A | 23 July 2021 | WO | 2020092205 | A1 | 07 May 2020 |
| | | | | EP | 3852683 | A1 | 28 July 2021 |
| | | | | EP | 3852683 | B1 | 29 May 2024 |
| | | | | CA | 3116158 | A1 | 07 May 2020 |
| | | | | US | 2023147439 | A1 | 11 May 2023 |
| | | | | US | 2020138573 | A1 | 07 May 2020 |
| | | | | US | 11517428 | B2 | 06 December 2022 |
| CN | 114681133 | A | 01 July 2022 | WO | 2022141789 | A1 | 07 July 2022 |
| | | | | CN | 215019728 | U | 07 December 2021 |
| US | 2021236688 | A1 | 05 August 2021 | EP | 3784175 | A1 | 03 March 2021 |
| | | | | WO | 2019210059 | A1 | 31 October 2019 |
| CN | 102764169 | A | 07 November 2012 | JP | 3196976 | U | 16 April 2015 |
| | | | | WO | 2013155970 | A1 | 24 October 2013 |
| | | | | DE | 212013000104 | U1 | 20 November 2014 |
| CN | 112107393 | A | 22 December 2020 | | None | | |
| US | 2016279297 | A1 | 29 September 2016 | JP | 2017213386 | A | 07 December 2017 |
| | | | | EP | 3060174 | A1 | 31 August 2016 |
| | | | | EP | 3060174 | B1 | 27 May 2020 |
| | | | | WO | 2015061431 | A1 | 30 April 2015 |
| | | | | JP | 2016539682 | A | 22 December 2016 |
| | | | | JP | 6220969 | B2 | 25 October 2017 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310945501

- CN 202310954713